# EUROPEAN PATENT SPECIFICATION

(11) **EP 3 265 583 B1**
(45) Date of publication and mention of the grant of the patent: **06.01.2021**
(21) Application number: 16709297.2
(22) Date of filing: 19.02.2016
(51) Int. Cl.: C12Q 1/689, C12Q 1/6883

(54) **METHOD OF DETECTING AVIAN NECROTIC ENTERITIS**
VERFAHREN ZUM NACHWEIS VON AVIÄRER NEKROTISCHER ENTERITIS
PROCÉDÉ DE DÉTECTION D'ENTÉRITE NÉCROTIQUE AVIAIRE

(30) Priority: 06.03.2015 US 201562129293 P; 29.06.2015 EP 15174270
(43) Date of publication of application: 10.01.2018
(60) Divisional application: 18173960.8
(73) Proprietor: Evonik Operations GmbH, 45128 Essen (DE)
(72) Inventor: IGWE, Emeka-Ignatius, 80333 München (DE); WALLMEIER, Holger, 65843 Sulzbach/Ts. (DE); PELZER, Stefan, 33335 Gütersloh (DE); FLÜGEL, Monika, 33803 Steinhagen (DE); BEKEL, Thomas, 33790 Halle (Westf.) (DE)
(74) Representative: Evonik Patent Association
(86) International application number: PCT/EP2016/053502
(87) International publication number: WO 2016/142146

(56) References cited:
- WO-A1-2008/148166
- WO-A1-2014/012168
- WO-A2-2005/016962
- MCCOURT M T ET AL: "Sandwich ELISA detection of Clostridium perfringens cells and alpha-toxin from field cases of necrotic enteritis of poultry", VETERINARY MICROBIOLOGY, ELSEVIER BV, NL, vol. 106, no. 3-4, 10 April 2005 (2005-04-10), pages 259-264, XP004791652, ISSN: 0378-1135, DOI: 10.1016/J.VETMIC.2004.12.023
- ANTHONY L. KEYBURN ET AL: "Association between avian necrotic enteritis and Clostridium perfringens strains expressing NetB toxin", VETERINARY RESEARCH, vol. 41, no. 2, March 2010 (2010-03), page 21, XP055001233, ISSN: 0928-4249, DOI: 10.1051/vetres/2009069
- DATABASE EMBL [Online] 5 December 2012 (2012-12-05), "Clostridium perfringens strain NE_10 plasmid pNetB-NE10, complete sequence.", XP002761708, retrieved from EBI accession no. EM_STD:JQ655731 Database accession no. JQ655731
- J. S. SCHOREY ET AL: "Exosomes and other extracellular vesicles in host-pathogen interactions", EMBO REPORTS, vol. 16, no. 1, 5 January 2015 (2015-01-05), pages 24-43, XP055211224, ISSN: 1469-221X, DOI: 10.15252/embr.201439363
- DATABASE WPI Week 201318 Thomson Scientific, London, GB; AN 2013-A47474 XP002744039, & CN 102 697 812 A (UNIV SHANDONG AGRIC) 3 October 2012 (2012-10-03)
- M. G. WISE ET AL: "Quantitative Detection of Clostridium perfringens in the Broiler Fowl Gastrointestinal Tract by Real-Time PCR", APPLIED AND ENVIRONMENTAL MICROBIOLOGY, vol. 71, no. 7, July 2005 (2005-07), pages 3911-3916, XP055232887, US ISSN: 0099-2240, DOI: 10.1128/AEM.71.7.3911-3916.2005
- KERRY K COOPER ET AL: "Diagnosing clostridial enteric disease in poultry", JOURNAL OF VETERINARY DIAGNOSTIC INVESTIGATION, vol. 25, 2013, pages 314-327, XP055211280,
- DION LEPP ET AL: "Identification of Novel Pathogenicity Loci in Clostridium perfringens Strains That Cause Avian Necrotic Enteritis", PLOS ONE, vol. 5, no. 5, May 2010 (2010-05), page e10795, XP055232293, DOI: 10.1371/journal.pone.0010795
- CATHY STAEDEL ET AL: "MicroRNAs and bacterial infection", CELLULAR MICROBIOLOGY, vol. 15, no. 9, 11 September 2013 (2013-09-11), pages 1496-1507, XP055211213, ISSN: 1462-5814, DOI: 10.1111/cmi.12159
- HUE DINH ET AL: "Modulation of microRNAs in two genetically disparate chicken lines showing different necrotic enteritis disease susceptibility", VETERINARY IMMUNOLOGY AND IMMUNOPATHOLOGY, vol. 159, no. 1-2, May 2014 (2014-05), pages 74-82, XP055211250, ISSN: 0165-2427, DOI: 10.1016/j.vetimm.2014.02.003 cited in the application

### SEQUENCE LISTING

<110> Evonik Degussa GmbH
<120> Method of detecting avian necrotic enteritis
<130> 201500092
<160> 17
<170> PatentIn version 3.5
<210> 1
   <211> 1884
   <212> DNA
   <213> Clostridium perfringens
<220>
   <221> CDS
   <222> (631)..(1884)
   <223> SAM gene
<400> 1
   cataaaaaat cactcctttt atatttttaa aaacattatc acttttttat taattatttt      60
   caatataatt agtataactg gtcaaaaatt atacataatt ggactgcagc acttatctta     120
   tatttaatac caagtttatc ttcttctatt atattaaaaa tattttctat ataattatat     180
   ttattattta cttaagtatt tagtaaatga attactatac ttctcttcat gattaagctt     240
   tatttaaatt tttatatata ttaattaact aaatttaata ctttatttta ctaaaaaaat     300
   tatatttgta tttaaataaa aaactactat tttttagtaa aataaagtat ttataaaaat     360
   attaatatac atttagttta tttttgatat atcaaaaact tttttatttt taaaaaaagt     420
   tgataattta atatagttga attttgctat aatatactaa agtttaaata aggagttgat     480
   aaacatgaaa tttaaagttt tagaaaaaaa ctttttaact atgtgtgagc caaatttttg     540
   ttgttcacca gtaacaaatt tataaaatat tatactaaaa aaactaactc tgatttcatc     600
   agggttagtt ttctatatgg aggttttaaa atg aaa aaa tca atg tat aat att      654
                                    Met Lys Lys Ser Met Tyr Asn Ile
                                    1               5
   tat ata caa gga aaa aaa gaa acc gca att ttc aac cta tta aca aga       702
   Tyr Ile Gln Gly Lys Lys Glu Thr Ala Ile Phe Asn Leu Leu Thr Arg
       10                  15                  20
   aat ata att gtt att tca aac aat gaa tat cta aat ttt gat aaa tta       750
   Asn Ile Ile Val Ile Ser Asn Asn Glu Tyr Leu Asn Phe Asp Lys Leu
   25                  30                  35                  40
   ata aat gat gaa gaa tgt aaa gaa caa aaa tct atg ggt ttt tct tta       798
   Ile Asn Asp Glu Glu Cys Lys Glu Gln Lys Ser Met Gly Phe Ser Leu
                   45                  50                  55
   gaa aat aat att aat gaa att gaa tta atg aaa tat act tta aat aaa       846
   Glu Asn Asn Ile Asn Glu Ile Glu Leu Met Lys Tyr Thr Leu Asn Lys
               60                  65                  70
   ggt aaa ttt tcc gaa aaa tca atg aca tta ttt tta tca ctc aca aga        894
   Gly Lys Phe Ser Glu Lys Ser Met Thr Leu Phe Leu Ser Leu Thr Arg
           75                  80                  85
   caa tgt aat tta aat tgc att tat tgc tat caa gat aga cga aaa aca        942
   Gln Cys Asn Leu Asn Cys Ile Tyr Cys Tyr Gln Asp Arg Arg Lys Thr
       90                  95                  100
   ttt gat gaa gga aat aat ttt tta aat aaa act gct tgg tta aaa att        990
   Phe Asp Glu Gly Asn Asn Phe Leu Asn Lys Thr Ala Trp Leu Lys Ile
   105                 110                 115                 120
   ttt gaa ttt tta aag aaa aaa tct att aat tta aat gaa tta cat ata       1038
   Phe Glu Phe Leu Lys Lys Lys Ser Ile Asn Leu Asn Glu Leu His Ile
                   125                 130                 135
   act tta ttt ggt gga gaa cct tta cta aat aaa tca att att tta caa       1086
   Thr Leu Phe Gly Gly Glu Pro Leu Leu Asn Lys Ser Ile Ile Leu Gln
               140                 145                 150
   att att gat gat tta aat tct tta aaa aat caa act cta aaa ata tat       1134
   Ile Ile Asp Asp Leu Asn Ser Leu Lys Asn Gln Thr Leu Lys Ile Tyr
           155                 160                 165
   ata aat tta ata act aat gga gtt ctt tta gat aaa aat ttt tgc gaa       1182
   Ile Asn Leu Ile Thr Asn Gly Val Leu Leu Asp Lys Asn Phe Cys Glu
       170                 175                 180
   aaa ata tat aaa tct ata aat tat att caa att aca ata gat ggt aat       1230
   Lys Ile Tyr Lys Ser Ile Asn Tyr Ile Gln Ile Thr Ile Asp Gly Asn
   185                 190                 195                 200
   aaa tca act cat aat caa tta aga gtt ttt cca gat ggt tct gga agc       1278
   Lys Ser Thr His Asn Gln Leu Arg Val Phe Pro Asp Gly Ser Gly Ser
                   205                 210                 215
   ttt gat att att tat aat aat tta aag gaa gta att aat cta ttt gaa       1326
   Phe Asp Ile Ile Tyr Asn Asn Leu Lys Glu Val Ile Asn Leu Phe Glu
               220                 225                 230
   aat aaa ata gaa tta aga att aat gta aat aaa gaa ata ata tta aat       1374
   Asn Lys Ile Glu Leu Arg Ile Asn Val Asn Lys Glu Ile Ile Leu Asn
           235                 240                 245
   tgc ttt tct tta ttt aaa aaa tta aag gaa gat tct tta aat aaa agt       1422
   Cys Phe Ser Leu Phe Lys Lys Leu Lys Glu Asp Ser Leu Asn Lys Ser
       250                 255                 260
   ata gcc ata aat tta cat cca att ttt gaa aat caa agc tct ttc gaa       1470
   Ile Ala Ile Asn Leu His Pro Ile Phe Glu Asn Gln Ser Ser Phe Glu
   265                 270                 275                 280
   tat ggc tgt aaa aag aat aca gac aaa gac ctt ata tta caa att aat       1518
   Tyr Gly Cys Lys Lys Asn Thr Asp Lys Asp Leu Ile Leu Gln Ile Asn
                   285                 290                 295
   gat tta tat aat tat tta tct aaa cat aat ttt aaa ttt aga aaa aac       1566
   Asp Leu Tyr Asn Tyr Leu Ser Lys His Asn Phe Lys Phe Arg Lys Asn
               300                 305                 310
   ttc ata gag ggt cca tgt ata gct aaa cat ata aat agt ttt gct ata       1614
   Phe Ile Glu Gly Pro Cys Ile Ala Lys His Ile Asn Ser Phe Ala Ile
           315                 320                 325
   gat gaa aat cta aat att tat aaa tgt cca ggc ttt tta tat tct aag           1662
   Asp Glu Asn Leu Asn Ile Tyr Lys Cys Pro Gly Phe Leu Tyr Ser Lys
       330                 335                 340
   tct aat gga ttt ata aat gat att gga gaa ata aac ttt tta gac tct           1710
   Ser Asn Gly Phe Ile Asn Asp Ile Gly Glu Ile Asn Phe Leu Asp Ser
   345                 350                 355                 360
   gat ttc ttt aaa gaa gta aat tct gaa caa aaa tgt att aat tct tgt           1758
   Asp Phe Phe Lys Glu Val Asn Ser Glu Gln Lys Cys Ile Asn Ser Cys
                   365                 370                 375
   ata tat gct cct att tgt tat ggt gga tgt act tgg caa aat aaa tgt           1806
   Ile Tyr Ala Pro Ile Cys Tyr Gly Gly Cys Thr Trp Gln Asn Lys Cys
               380                 385                 390
   aat aaa gag att tta gat tta act tta gaa tca caa tta aaa tct tat           1854
   Asn Lys Glu Ile Leu Asp Leu Thr Leu Glu Ser Gln Leu Lys Ser Tyr
           395                 400                 405
   tta ata tct aga tat aat ttt gaa ata taa                                   1884
   Leu Ile Ser Arg Tyr Asn Phe Glu Ile
       410                 415
<210> 2
   <211> 417
   <212> PRT
   <213> Clostridium perfringens
<400> 2
   Met Lys Lys Ser Met Tyr Asn Ile Tyr Ile Gln Gly Lys Lys Glu Thr
   1               5                   10                  15
   Ala Ile Phe Asn Leu Leu Thr Arg Asn Ile Ile Val Ile Ser Asn Asn
               20                  25                  30
   Glu Tyr Leu Asn Phe Asp Lys Leu Ile Asn Asp Glu Glu Cys Lys Glu
           35                  40                  45
   Gln Lys Ser Met Gly Phe Ser Leu Glu Asn Asn Ile Asn Glu Ile Glu
       50                  55                  60
   Leu Met Lys Tyr Thr Leu Asn Lys Gly Lys Phe Ser Glu Lys Ser Met
   65                  70                  75                  80
   Thr Leu Phe Leu Ser Leu Thr Arg Gln Cys Asn Leu Asn Cys Ile Tyr
                   85                  90                  95
   Cys Tyr Gln Asp Arg Arg Lys Thr Phe Asp Glu Gly Asn Asn Phe Leu
               100                 105                 110
   Asn Lys Thr Ala Trp Leu Lys Ile Phe Glu Phe Leu Lys Lys Lys Ser
               115                 120                 125
   Ile Asn Leu Asn Glu Leu His Ile Thr Leu Phe Gly Gly Glu Pro Leu
       130                 135                 140
   Leu Asn Lys Ser Ile Ile Leu Gln Ile Ile Asp Asp Leu Asn Ser Leu
   145                 150                 155                 160
   Lys Asn Gln Thr Leu Lys Ile Tyr Ile Asn Leu Ile Thr Asn Gly Val
                   165                 170                 175
   Leu Leu Asp Lys Asn Phe Cys Glu Lys Ile Tyr Lys Ser Ile Asn Tyr
               180                 185                 190
   Ile Gln Ile Thr Ile Asp Gly Asn Lys Ser Thr His Asn Gln Leu Arg
           195                 200                 205
   Val Phe Pro Asp Gly Ser Gly Ser Phe Asp Ile Ile Tyr Asn Asn Leu
       210                 215                 220
   Lys Glu Val Ile Asn Leu Phe Glu Asn Lys Ile Glu Leu Arg Ile Asn
   225                 230                 235                 240
   Val Asn Lys Glu Ile Ile Leu Asn Cys Phe Ser Leu Phe Lys Lys Leu
                   245                 250                 255
   Lys Glu Asp Ser Leu Asn Lys Ser Ile Ala Ile Asn Leu His Pro Ile
               260                 265                 270
   Phe Glu Asn Gln Ser Ser Phe Glu Tyr Gly Cys Lys Lys Asn Thr Asp
           275                 280                 285
   Lys Asp Leu Ile Leu Gln Ile Asn Asp Leu Tyr Asn Tyr Leu Ser Lys
       290                 295                 300
   His Asn Phe Lys Phe Arg Lys Asn Phe Ile Glu Gly Pro Cys Ile Ala
   305                 310                 315                 320
   Lys His Ile Asn Ser Phe Ala Ile Asp Glu Asn Leu Asn Ile Tyr Lys
                   325                 330                 335
   Cys Pro Gly Phe Leu Tyr Ser Lys Ser Asn Gly Phe Ile Asn Asp Ile
               340                 345                 350
   Gly Glu Ile Asn Phe Leu Asp Ser Asp Phe Phe Lys Glu Val Asn Ser
           355                 360                 365
   Glu Gln Lys Cys Ile Asn Ser Cys Ile Tyr Ala Pro Ile Cys Tyr Gly
       370                 375                 380
   Gly Cys Thr Trp Gln Asn Lys Cys Asn Lys Glu Ile Leu Asp Leu Thr
   385                 390                 395                 400
   Leu Glu Ser Gln Leu Lys Ser Tyr Leu Ile Ser Arg Tyr Asn Phe Glu
                   405                 410                 415
   Ile
<210> 3
   <211> 1679
   <212> DNA
   <213> Clostridium perfringens
<400> 3
   ttaaaaatag gttctaacaa ccatttctgt aaacatacca gtaggatttt ttctttggct     60
   taaaaagtat tcctttttat ctttaatctt ttcttttgac catctcatat aatcactctc    120
   ctaaaatatt atggttatta taataaaaag aaagtatcta aaatagatac tttcttagtt    180
   ataagttgaa atgtgaaaag ctataattat gatataaaaa ctcttaaaag atacacctta    240
   attataactt taaatttaat aaataaacag tatattactc tttatttttc ttaaaaaaat    300
   ttagtattgc atttttatct tttctacgtg gaaatgttaa atctaagaac tccatcattt    360
   tctttttagt ttgggttcat cctagtattg aagtattctt tagaatattt cctctctttt    420
   tgaaaatttc atcttcagta tctctaaatc ttgctttttc tgcagcattt attatacccg   480
   cagccatttt tatattatcc tggatttgtt tctcgtttaa tgaaccgtta aaattcctaa    540
   attcaactgt aggagctcta tctcttgcta tattagttaa gttaatacca taatatataa    600
   tttccttcag aaatttttgc tgccctcctt cttacatcct catcattatc catgttgaat    660
   cggatatatt tactttcatc ggccctagga ctaaatggag tggcataatg tctggcatta    720
   cttcttactc ttcctaaatc tccacctgca gctctataaa tacaatcctc atatacttct    780
   atagtcttaa aaaatcttct ccatctttgt cttgcagtat ctagtttatc catgtttata    840
   tgaacatggc caccgcacct ttgatctact ctagccccat gtcttttagc aacatcacat    900
   attattttta tttgttccca cgtttctgga gtatctttaa gaatagggga aactatttct   960
   ccgctggcat ctccatcgga tacggaccca tctctctcga gtttccactt acctggctca   1020
   cttcttgaat gataacttaa tcttctagga gagcttacta tacctagctc ataaagttct   1080
   atggctatag cattaacatc accaccaaca aactcaagtt caactccgaa tgtactattg   1140
   tttccgttta gtacgttttc gtactcatat tctatagaat cgtcattaat ttctgcaaaa   1200
   tcgttattga aatcagcttc attgtctgta taaaagtgat tatcgtcttg ttcttcccca   1260
   cgaatatttc tatttatttc atctcttata tttctagctc ttctagtagc ttctacattg   1320
   tttatagtat ctacttctgc aacttcgcct aaagcttttc tgaccgctat taagtgtctg   1380
   cagcattcat ttctataaac atatttgcca cattcacagc tattttcatt catatcaaca   1440
   atgtaagttc cttcttcagt tgaacttact tcatatcgat tattacccaa ggaaactata   1500
   tttatatctt caattctgtt tctggttcta gataaccctt gaacagttac aatgttatct   1560
   aatctaccat taacagcatt aactaaatct tctattattc cttcagaaag attagatcct   1620
   gagccatcaa ttaatccttc cgttgtattt gcaatagtat tgcttcttct tttaggcat    1679
<210> 4
   <211> 268
   <212> DNA
   <213> Clostridium perfringens
<400> 4
   gccgtgctag atggggagtt agcggtgccc tgtaacctgc aatccgctac agcaggatcg        60
   aagtcctcat agaggctcta acttgtggag tctgccctat gtaagtggtg ttgagagttg       120
   ggccccacgc aatggaaacc tgtgaacctc gtcaggtccg gaaggaagca gcgataagca       180
   gtcattttca tgtgccgtgg atacgtctgg cttgagctaa ctgcataggt aacgttcata       240
   agttattgtc gaagtgaggt gcacggtt                                          268
<210> 5
   <211> 3315
   <212> DNA
   <213> Clostridium perfringens
<220>
   <221> CDS
   <222> (1)..(3315)
   <223> ColA gene
<400> 5
   atg aag aaa aac tta aaa agg gga gag cta aca aaa ctg aag tta gtt          48
   Met Lys Lys Asn Leu Lys Arg Gly Glu Leu Thr Lys Leu Lys Leu Val
   1               5                   10                  15
   gaa aga tgg tca gct acc ttt act tta gca gca ttt att tta ttt aat          96
   Glu Arg Trp Ser Ala Thr Phe Thr Leu Ala Ala Phe Ile Leu Phe Asn
               20                  25                  30
   agc tca ttt aaa gta ttt gca gct gat aaa aag gta gag aat agt aat         144
   Ser Ser Phe Lys Val Phe Ala Ala Asp Lys Lys Val Glu Asn Ser Asn
           35                  40                  45
   aat gga cag att act aga gag att aat gct gat cag att tct aaa aca         192
   Asn Gly Gln Ile Thr Arg Glu Ile Asn Ala Asp Gln Ile Ser Lys Thr
       50                  55                  60
   gaa tta aat aat gag gta gct aca gac aat aat aga cca tta gga cct         240
   Glu Leu Asn Asn Glu Val Ala Thr Asp Asn Asn Arg Pro Leu Gly Pro
   65                  70                  75                  80
   agt att gct cca tca aga gca aga aac aac aag atc tat aca ttc gat          288
   Ser Ile Ala Pro Ser Arg Ala Arg Asn Asn Lys Ile Tyr Thr Phe Asp
                   85                  90                  95
   gaa ctt aac aga atg aat tat agt gat cta gtt gaa tta ata aaa aca          336
   Glu Leu Asn Arg Met Asn Tyr Ser Asp Leu Val Glu Leu Ile Lys Thr
               100                 105                 110
   ata agt tat gaa aac gta cca gac tta ttt aat ttt aat gat ggt tca          384
   Ile Ser Tyr Glu Asn Val Pro Asp Leu Phe Asn Phe Asn Asp Gly Ser
           115                 120                 125
   tat act ttc ttt agt aat aga gat cgt gta caa gct ata ata tat ggt          432
   Tyr Thr Phe Phe Ser Asn Arg Asp Arg Val Gln Ala Ile Ile Tyr Gly
       130                 135                 140
   cta gag gat agt gga aga act tat aca gca gat gat gat aag gga att          480
   Leu Glu Asp Ser Gly Arg Thr Tyr Thr Ala Asp Asp Asp Lys Gly Ile
   145                 150                 155                 160
   cca act tta gtt gag ttt tta aga gct gga tat tat tta gga ttt tat          528
   Pro Thr Leu Val Glu Phe Leu Arg Ala Gly Tyr Tyr Leu Gly Phe Tyr
                   165                 170                 175
   aat aaa caa tta tca tac cta aat aca cca cag tta aaa aat gag tgt          576
   Asn Lys Gln Leu Ser Tyr Leu Asn Thr Pro Gln Leu Lys Asn Glu Cys
               180                 185                 190
   tta cca gct atg aaa gcg att caa tat aat agt aat ttt aga tta gga          624
   Leu Pro Ala Met Lys Ala Ile Gln Tyr Asn Ser Asn Phe Arg Leu Gly
           195                 200                 205
   aca aag gcg caa gat gga gtt gtt gag gct tta gga aga ctt ata ggt          672
   Thr Lys Ala Gln Asp Gly Val Val Glu Ala Leu Gly Arg Leu Ile Gly
       210                 215                 220
   aat gct tca gca gat cca gaa gtt att aat aat tgc ata tat gtt tta          720
   Asn Ala Ser Ala Asp Pro Glu Val Ile Asn Asn Cys Ile Tyr Val Leu
   225                 230                 235                 240
   agt gat ttt aaa gat aat ata gat aag tat ggt tct aac tat agc aag          768
   Ser Asp Phe Lys Asp Asn Ile Asp Lys Tyr Gly Ser Asn Tyr Ser Lys
                   245                 250                 255
   gga aat gca gta ttc aac ctt atg aaa ggt att gat tat tat act aat          816
   Gly Asn Ala Val Phe Asn Leu Met Lys Gly Ile Asp Tyr Tyr Thr Asn
               260                 265                 270
   tca gta ata tac aat act aag gga tat gat gct aaa aac act gag ttc          864
   Ser Val Ile Tyr Asn Thr Lys Gly Tyr Asp Ala Lys Asn Thr Glu Phe
           275                 280                 285
   tat aat aga ata gat cca tat atg gaa aga tta gaa agt tta tgt aca          912
   Tyr Asn Arg Ile Asp Pro Tyr Met Glu Arg Leu Glu Ser Leu Cys Thr
       290                 295                 300
   ata ggt gat aag tta aat aat gat aat gct tgg ctt gta aat aat gcc          960
   Ile Gly Asp Lys Leu Asn Asn Asp Asn Ala Trp Leu Val Asn Asn Ala
   305                 310                 315                 320
   tta tat tac aca ggt aga atg ggt aag ttt aga gaa gac cca tca ata         1008
   Leu Tyr Tyr Thr Gly Arg Met Gly Lys Phe Arg Glu Asp Pro Ser Ile
                       325                 330                 335
   tct caa aga gct tta gaa aga gct atg aag gag tat cct tat tta tca        1056
   Ser Gln Arg Ala Leu Glu Arg Ala Met Lys Glu Tyr Pro Tyr Leu Ser
                   340                 345                 350
   tat caa tat att gaa gct gcc aat gat tta gat tta aat ttt ggt ggc        1104
   Tyr Gln Tyr Ile Glu Ala Ala Asn Asp Leu Asp Leu Asn Phe Gly Gly
               355                 360                 365
   aaa aat tca tca gga aat gat ata gat ttc aat aag ata aaa gca gat        1152
   Lys Asn Ser Ser Gly Asn Asp Ile Asp Phe Asn Lys Ile Lys Ala Asp
       370                 375                 380
   gca agg gaa aaa tat ctt cca aaa aca tat act ttt gat gat ggt aaa        1200
   Ala Arg Glu Lys Tyr Leu Pro Lys Thr Tyr Thr Phe Asp Asp Gly Lys
   385                 390                 395                 400
   ttt gta gta aaa gct ggt gat aaa gta aca gaa gag aag ata aaa aga        1248
   Phe Val Val Lys Ala Gly Asp Lys Val Thr Glu Glu Lys Ile Lys Arg
                       405                 410                 415
   tta tat tgg gct tca aag gaa gtt aag gct caa ttc atg aga gta gtt        1296
   Leu Tyr Trp Ala Ser Lys Glu Val Lys Ala Gln Phe Met Arg Val Val
                   420                 425                 430
   caa aat gat aag gct tta gaa gag gga aat cca gat gat att tta act        1344
   Gln Asn Asp Lys Ala Leu Glu Glu Gly Asn Pro Asp Asp Ile Leu Thr
               435                 440                 445
   gtt gtt att tat aac tca cca gaa gag tat aag tta aat cgt ata ata        1392
   Val Val Ile Tyr Asn Ser Pro Glu Glu Tyr Lys Leu Asn Arg Ile Ile
       450                 455                 460
   aat gga ttt agt act gat aat ggt ggt ata tat att gaa aac ata gga        1440
   Asn Gly Phe Ser Thr Asp Asn Gly Gly Ile Tyr Ile Glu Asn Ile Gly
   465                 470                 475                 480
   act ttc ttt act tat gaa aga aca cca gag gaa agt ata tat aca tta        1488
   Thr Phe Phe Thr Tyr Glu Arg Thr Pro Glu Glu Ser Ile Tyr Thr Leu
                       485                 490                 495
   gaa gaa tta ttc cgt cat gaa ttt act cac tat ctt caa ggt aga tat        1536
   Glu Glu Leu Phe Arg His Glu Phe Thr His Tyr Leu Gln Gly Arg Tyr
                   500                 505                 510
   gta gtt cct gga atg tgg ggg caa gga gaa ttc tat caa gag gga gtt        1584
   Val Val Pro Gly Met Trp Gly Gln Gly Glu Phe Tyr Gln Glu Gly Val
               515                 520                 525
   tta act tgg tat gaa gaa gga aca gca gag ttc ttt gca ggt tca act        1632
   Leu Thr Trp Tyr Glu Glu Gly Thr Ala Glu Phe Phe Ala Gly Ser Thr
       530                 535                 540
   aga act gat gga ata aaa cca aga aaa tca gtt aca caa ggg tta gct        1680
   Arg Thr Asp Gly Ile Lys Pro Arg Lys Ser Val Thr Gln Gly Leu Ala
   545                 550                 555                 560
   tac gat aga aat aat aga atg tct tta tat ggt gta tta cat gct aaa        1728
   Tyr Asp Arg Asn Asn Arg Met Ser Leu Tyr Gly Val Leu His Ala Lys
                       565                 570                 575
   tat ggc tca tgg gat ttc tat aat tat gga ttt gct cta tca aac tac        1776
   Tyr Gly Ser Trp Asp Phe Tyr Asn Tyr Gly Phe Ala Leu Ser Asn Tyr
               580                 585                 590
   atg tac aac aat aac atg gga atg ttt aat aag atg aca aat tac ata        1824
   Met Tyr Asn Asn Asn Met Gly Met Phe Asn Lys Met Thr Asn Tyr Ile
           595                 600                 605
   aag aat aat gat gta tct ggt tat aaa gat tat att gca tca atg agt        1872
   Lys Asn Asn Asp Val Ser Gly Tyr Lys Asp Tyr Ile Ala Ser Met Ser
       610                 615                 620
   agt gat tac gga tta aat gat aaa tat caa gac tat atg gat tct tta        1920
   Ser Asp Tyr Gly Leu Asn Asp Lys Tyr Gln Asp Tyr Met Asp Ser Leu
   625                 630                 635                 640
   tta aac aat att gat aac tta gat gtt cct tta gtt tca gat gag tat        1968
   Leu Asn Asn Ile Asp Asn Leu Asp Val Pro Leu Val Ser Asp Glu Tyr
                   645                 650                 655
   gta aat gga cat gaa gct aag gat ata aat gaa ata act aat gac ata        2016
   Val Asn Gly His Glu Ala Lys Asp Ile Asn Glu Ile Thr Asn Asp Ile
               660                 665                 670
   aaa gaa gtt tca aat ata aaa gat ctt tct agt aat gtt gaa aag tct        2064
   Lys Glu Val Ser Asn Ile Lys Asp Leu Ser Ser Asn Val Glu Lys Ser
           675                 680                 685
   caa ttc ttt act act tac gat atg aga gga aca tat gta ggg gga aga        2112
   Gln Phe Phe Thr Thr Tyr Asp Met Arg Gly Thr Tyr Val Gly Gly Arg
       690                 695                 700
   agt caa ggg gaa gaa aat gac tgg aaa gat atg aat tct aag tta aat        2160
   Ser Gln Gly Glu Glu Asn Asp Trp Lys Asp Met Asn Ser Lys Leu Asn
   705                 710                 715                 720
   gat atg tta aaa gaa tta tct aaa aag agc tgg aat ggt tat aaa act        2208
   Asp Met Leu Lys Glu Leu Ser Lys Lys Ser Trp Asn Gly Tyr Lys Thr
                   725                 730                 735
   gtt act gca tac ttt gta aac cat aaa gta gat gaa aat ggt aac tat        2256
   Val Thr Ala Tyr Phe Val Asn His Lys Val Asp Glu Asn Gly Asn Tyr
               740                 745                 750
   gtt tat gat gtt gta ttc cat gga atg aat aca gat aca aat act gat        2304
   Val Tyr Asp Val Val Phe His Gly Met Asn Thr Asp Thr Asn Thr Asp
           755                 760                 765
   gtt cat gtt aat aaa gag cct aag gct gtt ata aaa tct gat tct tca        2352
   Val His Val Asn Lys Glu Pro Lys Ala Val Ile Lys Ser Asp Ser Ser
       770                 775                 780
   gta ata gtt gaa gaa gaa att aat ttt gat gga aca gag tca aag gat        2400
   Val Ile Val Glu Glu Glu Ile Asn Phe Asp Gly Thr Glu Ser Lys Asp
   785                 790                 795                 800
   gaa gat ggt gaa atc aaa gct tat gaa tgg gac ttt gga gat gga gaa        2448
   Glu Asp Gly Glu Ile Lys Ala Tyr Glu Trp Asp Phe Gly Asp Gly Glu
                   805                 810                 815
   aaa tct aat gag gct aaa gcc act cat aag tat aat aaa act gga gaa        2496
   Lys Ser Asn Glu Ala Lys Ala Thr His Lys Tyr Asn Lys Thr Gly Glu
               820                 825                 830
   tat gaa gta aaa tta aca gtt aca gat aat aac ggt gga ata aat act        2544
   Tyr Glu Val Lys Leu Thr Val Thr Asp Asn Asn Gly Gly Ile Asn Thr
           835                 840                 845
   gaa agt aaa aag ata aaa gta gta gaa gat aaa cct gtt gaa gtt ata        2592
   Glu Ser Lys Lys Ile Lys Val Val Glu Asp Lys Pro Val Glu Val Ile
       850                 855                 860
   aat gaa agc gag cca aac aat gat ttt gaa aag gct aac caa ata gct        2640
   Asn Glu Ser Glu Pro Asn Asn Asp Phe Glu Lys Ala Asn Gln Ile Ala
   865                 870                 875                 880
   aaa tct aat atg tta gtt aag ggt act tta tca gaa gag gat tat tca        2688
   Lys Ser Asn Met Leu Val Lys Gly Thr Leu Ser Glu Glu Asp Tyr Ser
                   885                 890                 895
   gat aaa tat tat ttt gat gta gct aaa aaa ggc aat gtt aaa atc act        2736
   Asp Lys Tyr Tyr Phe Asp Val Ala Lys Lys Gly Asn Val Lys Ile Thr
               900                 905                 910
   ctt aat aat tta aat tca gta gga ata act tgg aca ctt tat aaa gag        2784
   Leu Asn Asn Leu Asn Ser Val Gly Ile Thr Trp Thr Leu Tyr Lys Glu
           915                 920                 925
   gga gac cta aac aat tat gtt tta tat gca act gga aat gat gga aca        2832
   Gly Asp Leu Asn Asn Tyr Val Leu Tyr Ala Thr Gly Asn Asp Gly Thr
       930                 935                 940
   gaa tta aag ggt gaa aag act tta gag cct gga aga tac tac tta agt        2880
   Glu Leu Lys Gly Glu Lys Thr Leu Glu Pro Gly Arg Tyr Tyr Leu Ser
   945                 950                 955                 960
   gta tat act tat gat aat caa tca gga gct tac aca gta aat gta aaa        2928
   Val Tyr Thr Tyr Asp Asn Gln Ser Gly Ala Tyr Thr Val Asn Val Lys
                   965                 970                 975
   gga aac ctt aaa aat gaa gtt aaa gaa aca gaa aag gat gct ata aaa        2976
   Gly Asn Leu Lys Asn Glu Val Lys Glu Thr Glu Lys Asp Ala Ile Lys
               980                 985                 990
   gaa gtt gaa aat aac aat gat ttt  gat aaa gct atg aag  gta gac agt      3024
   Glu Val Glu Asn Asn Asn Asp Phe  Asp Lys Ala Met Lys  Val Asp Ser
           995                 1000                1005
   aat agc  aaa ata gtt gga aca  tta agc aat gat gat  ctt aag gat         3069
   Asn Ser  Lys Ile Val Gly Thr  Leu Ser Asn Asp Asp  Leu Lys Asp
       1010                1015                1020
   att tat  agc ata gat ata caa  aat cca agt gac tta  aac ata gta         3114
   Ile Tyr  Ser Ile Asp Ile Gln  Asn Pro Ser Asp Leu  Asn Ile Val
       1025                1030                1035
   gtt gaa  aac tta gat aat ata  aaa atg aac tgg tta  tta tat tca         3159
   Val Glu  Asn Leu Asp Asn Ile  Lys Met Asn Trp Leu  Leu Tyr Ser
       1040                1045                1050
   gct gat  gat tta agt aac tat  gtg gat tac gct aat  gca gat gga         3204
   Ala Asp  Asp Leu Ser Asn Tyr  Val Asp Tyr Ala Asn  Ala Asp Gly
       1055                1060                1065
   aat aaa  tta agt aac act tgt  aag tta aat cca ggt  aaa tat tac         3249
   Asn Lys  Leu Ser Asn Thr Cys  Lys Leu Asn Pro Gly  Lys Tyr Tyr
       1070                1075                1080
   tta tgt  gtt tat caa ttt gaa  aac tca ggt act gga  aat tac aca         3294
   Leu Cys  Val Tyr Gln Phe Glu  Asn Ser Gly Thr Gly  Asn Tyr Thr
       1085                1090                1095
   gta aac  tta caa aac aaa taa                                           3315
   Val Asn  Leu Gln Asn Lys
       1100
<210> 6
   <211> 1104
   <212> PRT
   <213> Clostridium perfringens
<400> 6
   Met Lys Lys Asn Leu Lys Arg Gly Glu Leu Thr Lys Leu Lys Leu Val
   1               5                   10                  15
   Glu Arg Trp Ser Ala Thr Phe Thr Leu Ala Ala Phe Ile Leu Phe Asn
               20                  25                  30
   Ser Ser Phe Lys Val Phe Ala Ala Asp Lys Lys Val Glu Asn Ser Asn
           35                  40                  45
   Asn Gly Gln Ile Thr Arg Glu Ile Asn Ala Asp Gln Ile Ser Lys Thr
       50                  55                  60
   Glu Leu Asn Asn Glu Val Ala Thr Asp Asn Asn Arg Pro Leu Gly Pro
   65                  70                  75                  80
   Ser Ile Ala Pro Ser Arg Ala Arg Asn Asn Lys Ile Tyr Thr Phe Asp
                   85                  90                  95
   Glu Leu Asn Arg Met Asn Tyr Ser Asp Leu Val Glu Leu Ile Lys Thr
               100                 105                 110
   Ile Ser Tyr Glu Asn Val Pro Asp Leu Phe Asn Phe Asn Asp Gly Ser
           115                 120                 125
   Tyr Thr Phe Phe Ser Asn Arg Asp Arg Val Gln Ala Ile Ile Tyr Gly
       130                 135                 140
   Leu Glu Asp Ser Gly Arg Thr Tyr Thr Ala Asp Asp Asp Lys Gly Ile
   145                 150                 155                 160
   Pro Thr Leu Val Glu Phe Leu Arg Ala Gly Tyr Tyr Leu Gly Phe Tyr
                   165                 170                 175
   Asn Lys Gln Leu Ser Tyr Leu Asn Thr Pro Gln Leu Lys Asn Glu Cys
               180                 185                 190
   Leu Pro Ala Met Lys Ala Ile Gln Tyr Asn Ser Asn Phe Arg Leu Gly
           195                 200                 205
   Thr Lys Ala Gln Asp Gly Val Val Glu Ala Leu Gly Arg Leu Ile Gly
       210                 215                 220
   Asn Ala Ser Ala Asp Pro Glu Val Ile Asn Asn Cys Ile Tyr Val Leu
   225                 230                 235                 240
   Ser Asp Phe Lys Asp Asn Ile Asp Lys Tyr Gly Ser Asn Tyr Ser Lys
                   245                 250                 255
   Gly Asn Ala Val Phe Asn Leu Met Lys Gly Ile Asp Tyr Tyr Thr Asn
               260                 265                 270
   Ser Val Ile Tyr Asn Thr Lys Gly Tyr Asp Ala Lys Asn Thr Glu Phe
           275                 280                 285
   Tyr Asn Arg Ile Asp Pro Tyr Met Glu Arg Leu Glu Ser Leu Cys Thr
       290                 295                 300
   Ile Gly Asp Lys Leu Asn Asn Asp Asn Ala Trp Leu Val Asn Asn Ala
   305                 310                 315                 320
   Leu Tyr Tyr Thr Gly Arg Met Gly Lys Phe Arg Glu Asp Pro Ser Ile
                   325                 330                 335
   Ser Gln Arg Ala Leu Glu Arg Ala Met Lys Glu Tyr Pro Tyr Leu Ser
               340                 345                 350
   Tyr Gln Tyr Ile Glu Ala Ala Asn Asp Leu Asp Leu Asn Phe Gly Gly
           355                 360                 365
   Lys Asn Ser Ser Gly Asn Asp Ile Asp Phe Asn Lys Ile Lys Ala Asp
       370                 375                 380
   Ala Arg Glu Lys Tyr Leu Pro Lys Thr Tyr Thr Phe Asp Asp Gly Lys
   385                 390                 395                 400
   Phe Val Val Lys Ala Gly Asp Lys Val Thr Glu Glu Lys Ile Lys Arg
                   405                 410                 415
   Leu Tyr Trp Ala Ser Lys Glu Val Lys Ala Gln Phe Met Arg Val Val
               420                 425                 430
   Gln Asn Asp Lys Ala Leu Glu Glu Gly Asn Pro Asp Asp Ile Leu Thr
           435                 440                 445
   Val Val Ile Tyr Asn Ser Pro Glu Glu Tyr Lys Leu Asn Arg Ile Ile
       450                 455                 460
   Asn Gly Phe Ser Thr Asp Asn Gly Gly Ile Tyr Ile Glu Asn Ile Gly
   465                 470                 475                 480
   Thr Phe Phe Thr Tyr Glu Arg Thr Pro Glu Glu Ser Ile Tyr Thr Leu
                   485                 490                 495
   Glu Glu Leu Phe Arg His Glu Phe Thr His Tyr Leu Gln Gly Arg Tyr
               500                 505                 510
   Val Val Pro Gly Met Trp Gly Gln Gly Glu Phe Tyr Gln Glu Gly Val
           515                 520                 525
   Leu Thr Trp Tyr Glu Glu Gly Thr Ala Glu Phe Phe Ala Gly Ser Thr
       530                 535                 540
   Arg Thr Asp Gly Ile Lys Pro Arg Lys Ser Val Thr Gln Gly Leu Ala
   545                 550                 555                 560
   Tyr Asp Arg Asn Asn Arg Met Ser Leu Tyr Gly Val Leu His Ala Lys
                   565                 570                 575
   Tyr Gly Ser Trp Asp Phe Tyr Asn Tyr Gly Phe Ala Leu Ser Asn Tyr
               580                 585                 590
   Met Tyr Asn Asn Asn Met Gly Met Phe Asn Lys Met Thr Asn Tyr Ile
           595                 600                 605
   Lys Asn Asn Asp Val Ser Gly Tyr Lys Asp Tyr Ile Ala Ser Met Ser
       610                 615                 620
   Ser Asp Tyr Gly Leu Asn Asp Lys Tyr Gln Asp Tyr Met Asp Ser Leu
   625                 630                 635                 640
   Leu Asn Asn Ile Asp Asn Leu Asp Val Pro Leu Val Ser Asp Glu Tyr
                   645                 650                 655
   Val Asn Gly His Glu Ala Lys Asp Ile Asn Glu Ile Thr Asn Asp Ile
               660                 665                 670
   Lys Glu Val Ser Asn Ile Lys Asp Leu Ser Ser Asn Val Glu Lys Ser
           675                 680                 685
   Gln Phe Phe Thr Thr Tyr Asp Met Arg Gly Thr Tyr Val Gly Gly Arg
   690 695 700
   Ser Gln Gly Glu Glu Asn Asp Trp Lys Asp Met Asn Ser Lys Leu Asn
   705 710 715 720
   Asp Met Leu Lys Glu Leu Ser Lys Lys Ser Trp Asn Gly Tyr Lys Thr
   725 730 735
   Val Thr Ala Tyr Phe Val Asn His Lys Val Asp Glu Asn Gly Asn Tyr
   740 745 750
   Val Tyr Asp Val Val Phe His Gly Met Asn Thr Asp Thr Asn Thr Asp
   755 760 765
   Val His Val Asn Lys Glu Pro Lys Ala Val Ile Lys Ser Asp Ser Ser
   770 775 780
   Val Ile Val Glu Glu Glu Ile Asn Phe Asp Gly Thr Glu Ser Lys Asp
   785 790 795 800
   Glu Asp Gly Glu Ile Lys Ala Tyr Glu Trp Asp Phe Gly Asp Gly Glu
   805 810 815
   Lys Ser Asn Glu Ala Lys Ala Thr His Lys Tyr Asn Lys Thr Gly Glu
   820 825 830
   Tyr Glu Val Lys Leu Thr Val Thr Asp Asn Asn Gly Gly Ile Asn Thr
   835 840 845
   Glu Ser Lys Lys Ile Lys Val Val Glu Asp Lys Pro Val Glu Val Ile
   850 855 860
   Asn Glu Ser Glu Pro Asn Asn Asp Phe Glu Lys Ala Asn Gln Ile Ala
   865 870 875 880
   Lys Ser Asn Met Leu Val Lys Gly Thr Leu Ser Glu Glu Asp Tyr Ser
   885 890 895
   Asp Lys Tyr Tyr Phe Asp Val Ala Lys Lys Gly Asn Val Lys Ile Thr
   900 905 910
   Leu Asn Asn Leu Asn Ser Val Gly Ile Thr Trp Thr Leu Tyr Lys Glu
   915 920 925
   Gly Asp Leu Asn Asn Tyr Val Leu Tyr Ala Thr Gly Asn Asp Gly Thr
   930 935 940
   Glu Leu Lys Gly Glu Lys Thr Leu Glu Pro Gly Arg Tyr Tyr Leu Ser
   945                 950                 955                 960
   Val Tyr Thr Tyr Asp Asn Gln Ser Gly Ala Tyr Thr Val Asn Val Lys
                   965                 970                 975
   Gly Asn Leu Lys Asn Glu Val Lys Glu Thr Glu Lys Asp Ala Ile Lys
               980                 985                 990
   Glu Val Glu Asn Asn Asn Asp Phe  Asp Lys Ala Met Lys  Val Asp Ser
           995                 1000                1005
   Asn Ser  Lys Ile Val Gly Thr  Leu Ser Asn Asp Asp  Leu Lys Asp
       1010                 1015                 1020
   Ile Tyr  Ser Ile Asp Ile Gln  Asn Pro Ser Asp Leu  Asn Ile Val
       1025                 1030                 1035
   Val Glu  Asn Leu Asp Asn Ile  Lys Met Asn Trp Leu  Leu Tyr Ser
       1040                 1045                 1050
   Ala Asp  Asp Leu Ser Asn Tyr  Val Asp Tyr Ala Asn  Ala Asp Gly
       1055                 1060                 1065
   Asn Lys  Leu Ser Asn Thr Cys  Lys Leu Asn Pro Gly  Lys Tyr Tyr
       1070                 1075                 1080
   Leu Cys  Val Tyr Gln Phe Glu  Asn Ser Gly Thr Gly  Asn Tyr Thr
       1085                 1090                 1095
   Val Asn  Leu Gln Asn Lys
       1100
<210> 7
   <211> 1001
   <212> DNA
   <213> Clostridium perfringens
<400> 7
   tcaaaataga gtgcttttac ttataaaact taatatattt attcctaaaa tatcagataa      60
   aattaagact atgttatata taatgagaag gaaattaaat atttttagta ttttatttat     120
   tatcttttca gtttttgatc cagttttaat tttggctatt ttaaagttag ttttccaagg     180
   atagaaaagt tctattcctt ctgaagttaa aaaatctaaa actaagtggg atatatatcc     240
   agctataaat cctattgaaa ttattaataa tattatgttg aagttactaa catttaaaat     300
   aatatataag gttaatacta ttaaagatag acatagtaaa ctatgagtaa agccacgatg     360
   tctacattta ctacctatat gctttgaaag aaaaggatac atttgactaa ttgaagattt     420
   tctttgatct atatcaggaa atagagatcc tatataagaa aaatgaaaaa ataatgctat     480
   taataaaatt ttataagcta tatcatatgg aaataagaac aggcttaaaa ttgtatttaa     540
   cattattaaa gaaatgcata cgcctccagt tgaatgagtt tcttttgtca taaaatctcc     600
   tttaaaaata attatcatta gtttatatta aaaatattat cataatattc attttacata     660
   aagaagaaaa aaataaaaat cctaaaaata gagtggacag ttctatttct aggatcaata     720
   gttatggtgg taattttatg taaggttttt tactaaatta aagtaaaatc attaataagt     780
   aattatttat ttttaattac aattatggaa tatgcaattg aaaatattga tattaaagca     840
   agtatgggac ttataatatt tgaaacaact tctaaagatg taaaataaga tactctttgt     900
   tcaatatata aagctatgct atttttaagt attaagggag aaaaaagagt tattaatgta     960
   attaaaattg cacttgttaa aatccttttt gtatgtttca a                        1001
<210> 8
   <211> 960
   <212> DNA
   <213> Clostridium perfringens
<220>
   <221> misc_feature
   <222> (527)..(527)
   <223> n is a, c, g, t or u
<400> 8
   ttacagataa tattctattt tatgatcttg ccaatttatt ttaaacataa attcgttata      60
   ttcacttgtt gacgaaagtt tgtttgttcc tcgccattga gtagtttccc aatttaaaat     120
   atagtcatta tcaaatcttt gatattcaac tattattaca gattctttag catttttagg     180
   tgctgttaat gctaaagcca tattgggtga aaatccacca gaaattaatg ttgataaatc     240
   tctatcatct gtgaaatttt tatcaccatt attatacaat cttgatttca tgaataattg     300
   atttccataa atagcatgat aagaatctat attataaccg tccttagtct caacaaattt     360
   tatatcccat gatgctaaat ttgcatcatc ttttctttga attgttctaa aatcaggttg     420
   ttcatagctt atagtatttt ggacattata tgaagcattt attccagcac cagcagtttt     480
   tccttcaaca gatatattac cgcctataga ataaccaatt gaattanata catctttttt     540
   atctatagtt ttaggaatag aatttgctat attattattt acatcagcac tttttacatt     600
   aattctataa gtttcaggcc atttcatttt tccgtaatat ttagaaccaa aaatctgttt     660
   atcagaaggt ataaatcctt ctaaatttaa taaagcagtt ttcttatcag aatgaggatc     720
   ttcaataaat gttccactta aagttgcctt ccaaccttta tgtgaagcgg tatcactaga     780
   agtatattta atagcttcct ttccattttc ttttattatt tctccactta gatttttcaa     840
   ctcaattttg tttatgtcat ttaattcact tgcaaaaact tgagtttgag ttgaaaaaag     900
   gcttgtacta attacacttg taagaactag tgtaattgaa ataattttta atcttttcaa     960
<210> 9
   <211> 562
   <212> DNA
   <213> Clostridium perfringens
<400> 9
   tgtttatcat cccttttctc tcatttttat atactcataa atatatcata aaaagcaaaa      60
   tctatccata atatatttaa aaaatgagta tatagttttt aataaaatta tatttaatta     120
   tgtataaaaa tcataagtta aattgctcta tataatattt ccaaataaga ttaattttat     180
   gaaatagaaa tgaaaaaagt tctgcaaagt gcagaacttt taatttatga ataaacttgc     240
   gttaattatc aattatctat tcattttttg agcttttctt gcttttctgc aagctgggca     300
   tctaactgga tcgttttcaa atcctttttc tttgaagaat tcttgttctc ctacagtgaa     360
   tacgaattct tttccacagt ctttacaaac taaattttta tcttccattt cttttacctc     420
   ctgtaaaatt tactaggtac gtttgagatc tatactataa aactaataaa ataaatttta     480
   caggacagaa ataaagtaca tatctactaa attttatttt atatattgat tatatcatat     540
   agaaaaaaga atacaatact tt                                              562
<210> 10
   <211> 84
   <212> RNA
   <213> Gallus gallus
<400> 10
   gucugucaua cucuagcagc acguaaauau ugguguuaaa acuguaaaua ucuccaguau 60
   uaacugugcu gcugaaguaa ggcu 84
<210> 11
   <211> 68
   <212> RNA
   <213> Gallus gallus
<400> 11
   cuccggugcc uacugagcug auaucaguuc ugauuuuaca uacuggcuca guucagcagg 60
   aacaggag 68
<210> 12
   <211> 63
   <212> RNA
   <213> Gallus gallus
<400> 12
   uguuaaugcu aaucgugaua gggguuuuua ccucugaaug acuccuacau guuagcauua 60
   aca 63
<210> 13
   <211> 76
   <212> RNA
   <213> Gallus gallus
<400> 13
   gagaugacau gcuucuuuau auccccauau ggauuaggcu gcuauggaau guaaggaagu     60
   gugugguuuc agggag 76
<210> 14
   <211> 77
   <212> PRT
   <213> Clostridium perfringens
<400> 14
   Met Lys Glu Ile Ile Tyr Tyr Gly Ile Asn Leu Thr Asn Ile Ala Arg
   1               5                   10                  15
   Asp Arg Ala Pro Thr Val Glu Phe Arg Asn Phe Asn Gly Ser Leu Asn
               20                  25                  30
   Glu Lys Gln Ile Gln Asp Asn Ile Lys Met Ala Ala Gly Ile Ile Asn
           35                  40                  45
   Ala Ala Glu Lys Ala Arg Phe Arg Asp Thr Glu Asp Glu Ile Phe Lys
       50                  55                  60
   Lys Arg Gly Asn Ile Leu Lys Asn Thr Ser Ile Leu Gly
   65                  70                  75
<210> 15
   <211> 196
   <212> PRT
   <213> Clostridium perfringens
<400> 15
   Met Thr Lys Glu Thr His Ser Thr Gly Gly Val Cys Ile Ser Leu Ile
   1               5                   10                  15
   Met Leu Asn Thr Ile Leu Ser Leu Phe Leu Phe Pro Tyr Asp Ile Ala
               20                  25                  30
   Tyr Lys Ile Leu Leu Ile Ala Leu Phe Phe His Phe Ser Tyr Ile Gly
           35                  40                  45
   Ser Leu Phe Pro Asp Ile Asp Gln Arg Lys Ser Ser Ile Ser Gln Met
       50                  55                  60
   Tyr Pro Phe Leu Ser Lys His Ile Gly Ser Lys Cys Arg His Arg Gly
   65                  70                  75                  80
   Phe Thr His Ser Leu Leu Cys Leu Ser Leu Ile Val Leu Thr Leu Tyr
                   85                  90                  95
   Ile Ile Leu Asn Val Ser Asn Phe Asn Ile Ile Leu Leu Ile Ile Ser
               100                 105                 110
   Ile Gly Phe Ile Ala Gly Tyr Ile Ser His Leu Val Leu Asp Phe Leu
           115                 120                 125
   Thr Ser Glu Gly Ile Glu Leu Phe Tyr Pro Trp Lys Thr Asn Phe Lys
       130                 135                 140
   Ile Ala Lys Ile Lys Thr Gly Ser Lys Thr Glu Lys Ile Ile Asn Lys
   145                 150                 155                 160
   Ile Leu Lys Ile Phe Asn Phe Leu Leu Ile Ile Tyr Asn Ile Val Leu
                   165                 170                 175
   Ile Leu Ser Asp Ile Leu Gly Ile Asn Ile Leu Ser Phe Ile Ser Lys
               180                 185                 190
   Ser Thr Leu Phe
           195
<210> 16
   <211> 5628
   <212> DNA
   <213> Clostridium perfringens
<220>
   <221> CDS
   <222> (673)..(5628)
   <223> TpeL gene
<400> 16
   gatcttaatg aacctaatac agatatattt gtttatgatt taatagacaa gctttcagaa      60
   aaagaaagaa aaataattaa atataaatac atacatggaa aaagtgatgt tgaaataggg     120
   aaattactta attattctag acagcacgta aaccaaataa aaaatagagc attaaaaaat     180
   ttgaagaaat ttttagagga gtgatttaat ctaaatatta aaattaaata tttataggag     240
   gttaattgtg gattcagaac tgtttaaaat tatggctact caaggagctt ttgctttact     300
   attttcctac ttgttatttt atgtattaaa agaaaatagc aaaagagaag aaaattatca     360
   aaatataatt aaggagctta cagaattact accaattata aaaagtgatg ttgaagacat     420
   aaaaaataaa ttgaataata attagaaaaa atttataaaa aatataaatt ttaaattttt      480
   atttacaaaa tttaaaatta gaactcttta tataatgtat aagaaattta attctaagta      540
   tataaaatac taaaggaggg ttaagaagaa taaaatttga aaggaaaaaa tttgtgcaaa      600
   ttcaaattta taattagatt gaagggagga tttgatgtaa atataaatta agttttacat      660
   caacaataat at atg ggg tta atg tca aaa gaa caa tta att ata tta gca      711
                 Met Gly Leu Met Ser Lys Glu Gln Leu Ile Ile Leu Ala
                 1               5                   10
   aaa aat tca agt cca aaa gaa gga gaa tat aaa aaa att tta gaa tta        759
   Lys Asn Ser Ser Pro Lys Glu Gly Glu Tyr Lys Lys Ile Leu Glu Leu
       15                  20                  25
   cta gat gaa tat aat tta tta aat aat tct gtt gaa aaa aat agt ata        807
   Leu Asp Glu Tyr Asn Leu Leu Asn Asn Ser Val Glu Lys Asn Ser Ile
   30                  35                  40                  45
   gat tta tat tta aaa ctt aat gaa tta tct aaa tca att gat att tat        855
   Asp Leu Tyr Leu Lys Leu Asn Glu Leu Ser Lys Ser Ile Asp Ile Tyr
                   50                  55                  60
   tta aaa aaa tat aag aat tca aag aga aat aat gca tta tat caa tta        903
   Leu Lys Lys Tyr Lys Asn Ser Lys Arg Asn Asn Ala Leu Tyr Gln Leu
               65                  70                  75
   aaa tct gat tta aca aag gaa gtt att gaa ata aaa gat act aat tta        951
   Lys Ser Asp Leu Thr Lys Glu Val Ile Glu Ile Lys Asp Thr Asn Leu
           80                  85                  90
   aaa cct ttg gaa aaa aat att cat ttt gtt tgg gtt gga gga atg ata        999
   Lys Pro Leu Glu Lys Asn Ile His Phe Val Trp Val Gly Gly Met Ile
       95                  100                 105
   aat aat ata tct att gat tat ata aat caa tgg aaa gat att aat agc       1047
   Asn Asn Ile Ser Ile Asp Tyr Ile Asn Gln Trp Lys Asp Ile Asn Ser
   110                 115                 120                 125
   gat tat gaa act att ata tgg tat gat agt gaa gct ttg tta gta aat       1095
   Asp Tyr Glu Thr Ile Ile Trp Tyr Asp Ser Glu Ala Leu Leu Val Asn
                   130                 135                 140
   ata tta aaa aaa gct ata ata gac tca tca aat aaa gaa gtt tta act       1143
   Ile Leu Lys Lys Ala Ile Ile Asp Ser Ser Asn Lys Glu Val Leu Thr
               145                 150                 155
   aaa tat gaa tca gta tta aat gat aat agc ttt gat tca aat aaa ttt       1191
   Lys Tyr Glu Ser Val Leu Asn Asp Asn Ser Phe Asp Ser Asn Lys Phe
           160                 165                 170
   tat aga gaa aga atg gaa gtt att ttt aga aag caa aaa gag ttt aat       1239
   Tyr Arg Glu Arg Met Glu Val Ile Phe Arg Lys Gln Lys Glu Phe Asn
       175                 180                 185
   aat tat tat aat aca aat gat aac tat act aaa tct tta aat gat gta       1287
   Asn Tyr Tyr Asn Thr Asn Asp Asn Tyr Thr Lys Ser Leu Asn Asp Val
   190                 195                 200                 205
   att aaa gtt tat tta ata gaa aaa tat tta aaa act gat gaa gaa tta       1335
   Ile Lys Val Tyr Leu Ile Glu Lys Tyr Leu Lys Thr Asp Glu Glu Leu
                   210                 215                 220
   gaa aaa tat att aat gaa agt aaa gaa gtt ttt aag gct aat gga gct        1383
   Glu Lys Tyr Ile Asn Glu Ser Lys Glu Val Phe Lys Ala Asn Gly Ala
               225                 230                 235
   aaa gat ata aga gaa tac gat ata cta gat gat gtt gaa tta aaa tca        1431
   Lys Asp Ile Arg Glu Tyr Asp Ile Leu Asp Asp Val Glu Leu Lys Ser
           240                 245                 250
   ata tat gag caa gag tta tta atg aga ttt aat tta gca tct gca tca        1479
   Ile Tyr Glu Gln Glu Leu Leu Met Arg Phe Asn Leu Ala Ser Ala Ser
       255                 260                 265
   gat att att aga gtg att gta tta aat aaa tta ggt ggg att tat tta        1527
   Asp Ile Ile Arg Val Ile Val Leu Asn Lys Leu Gly Gly Ile Tyr Leu
   270                 275                 280                 285
   gat gta gat gtg ctt cct gga ata aaa aaa cat att ttt aaa gat att        1575
   Asp Val Asp Val Leu Pro Gly Ile Lys Lys His Ile Phe Lys Asp Ile
                   290                 295                 300
   aat aaa cct aca aat att tct gaa aat aaa tgg caa atg att caa tta        1623
   Asn Lys Pro Thr Asn Ile Ser Glu Asn Lys Trp Gln Met Ile Gln Leu
               305                 310                 315
   gaa act att atg aaa tat aaa caa tat ata aaa gga tat aca gaa aat        1671
   Glu Thr Ile Met Lys Tyr Lys Gln Tyr Ile Lys Gly Tyr Thr Glu Asn
           320                 325                 330
   tca ttt aaa aat tta cca agt gat ttg cag gaa atg tta caa gaa aaa        1719
   Ser Phe Lys Asn Leu Pro Ser Asp Leu Gln Glu Met Leu Gln Glu Lys
       335                 340                 345
   gtt gtt gaa aaa aat ctt aaa agt gat ata ttt cag aga tta ggg gat        1767
   Val Val Glu Lys Asn Leu Lys Ser Asp Ile Phe Gln Arg Leu Gly Asp
   350                 355                 360                 365
   ata ttt att tca gaa tta gat aca aaa ata gct ttt atg ttt ggg aaa        1815
   Ile Phe Ile Ser Glu Leu Asp Thr Lys Ile Ala Phe Met Phe Gly Lys
                   370                 375                 380
   att gct aat caa gta tta ata tct aaa aaa aat tca tac tca tta aat        1863
   Ile Ala Asn Gln Val Leu Ile Ser Lys Lys Asn Ser Tyr Ser Leu Asn
               385                 390                 395
   tta ata att aat caa atc aag aat aga tat aat att att aat aaa tgt        1911
   Leu Ile Ile Asn Gln Ile Lys Asn Arg Tyr Asn Ile Ile Asn Lys Cys
           400                 405                 410
   tta tct tcg gct att gaa aaa gga tca aat ttt aat aat aca gtt gat        1959
   Leu Ser Ser Ala Ile Glu Lys Gly Ser Asn Phe Asn Asn Thr Val Asp
       415                 420                 425
   ata ttc att caa caa tta aat gaa ttc tat gtt aat gaa ggc ttt ttt        2007
   Ile Phe Ile Gln Gln Leu Asn Glu Phe Tyr Val Asn Glu Gly Phe Phe
   430                 435                 440                 445
   gtt agt aaa gta atg gga tat tta ggt gat gga tat atg cct gat atg        2055
   Val Ser Lys Val Met Gly Tyr Leu Gly Asp Gly Tyr Met Pro Asp Met
                   450                 455                 460
   aga gcg aca ctt aat ata agt ggt cca gga ata tat aca gct gct tat        2103
   Arg Ala Thr Leu Asn Ile Ser Gly Pro Gly Ile Tyr Thr Ala Ala Tyr
                   465                 470                 475
   tat gat tta tta tat ttt aat gag agg tca tta aat cct caa att tta        2151
   Tyr Asp Leu Leu Tyr Phe Asn Glu Arg Ser Leu Asn Pro Gln Ile Leu
               480                 485                 490
   caa gaa gat ttg aaa tat ttt gaa gtt cca caa gct cta ata tca caa        2199
   Gln Glu Asp Leu Lys Tyr Phe Glu Val Pro Gln Ala Leu Ile Ser Gln
       495                 500                 505
   caa aca gaa caa gaa ata aat agt tca tgg aca ttt aat caa gtt aaa        2247
   Gln Thr Glu Gln Glu Ile Asn Ser Ser Trp Thr Phe Asn Gln Val Lys
   510                 515                 520                 525
   tca caa ata gaa tat aaa aag cta gta gaa aaa tat act aat aaa tca        2295
   Ser Gln Ile Glu Tyr Lys Lys Leu Val Glu Lys Tyr Thr Asn Lys Ser
                   530                 535                 540
   tta agt gaa aat gat aaa cta aat ttt aat gag aat aaa atc att gat        2343
   Leu Ser Glu Asn Asp Lys Leu Asn Phe Asn Glu Asn Lys Ile Ile Asp
               545                 550                 555
   aaa gta gaa tta cta aat cga att aat agt aat aat tta att aat ttt        2391
   Lys Val Glu Leu Leu Asn Arg Ile Asn Ser Asn Asn Leu Ile Asn Phe
           560                 565                 570
   gat gat aaa gaa tac tta aga tat ata att caa tta caa ggt gat aaa        2439
   Asp Asp Lys Glu Tyr Leu Arg Tyr Ile Ile Gln Leu Gln Gly Asp Lys
       575                 580                 585
   gta agt tat gag gct gca ata aat tta ttt att aaa aat cct tca aat        2487
   Val Ser Tyr Glu Ala Ala Ile Asn Leu Phe Ile Lys Asn Pro Ser Asn
   590                 595                 600                 605
   tca ata cta gtt caa gaa att aat aat ata agc tac tat ttt aat tca        2535
   Ser Ile Leu Val Gln Glu Ile Asn Asn Ile Ser Tyr Tyr Phe Asn Ser
                   610                 615                 620
   gaa tat aaa tca ata gat tct ata caa ttt gat aat att cct gaa ata        2583
   Glu Tyr Lys Ser Ile Asp Ser Ile Gln Phe Asp Asn Ile Pro Glu Ile
               625                 630                 635
   tta aaa ggt aaa aat aaa att aaa tta aca ttt ata ggt cat gga gaa        2631
   Leu Lys Gly Lys Asn Lys Ile Lys Leu Thr Phe Ile Gly His Gly Glu
           640                 645                 650
   gaa gaa ttt aat aca gag aga ttt gct tca tta aca gta aag gag ttt        2679
   Glu Glu Phe Asn Thr Glu Arg Phe Ala Ser Leu Thr Val Lys Glu Phe
       655                 660                 665
   tca aaa aaa ata tat aaa gtt tta gat atg ata aag agc aat acg aat        2727
   Ser Lys Lys Ile Tyr Lys Val Leu Asp Met Ile Lys Ser Asn Thr Asn
   670                 675                 680                 685
   gtt aaa gaa att caa ata gat ttg tta gga tgt aat atg ttt tca tat        2775
   Val Lys Glu Ile Gln Ile Asp Leu Leu Gly Cys Asn Met Phe Ser Tyr
                   690                 695                 700
   aat ata aat gta gaa gaa aca tat cct ggg aaa tta tta aaa gta gta        2823
   Asn Ile Asn Val Glu Glu Thr Tyr Pro Gly Lys Leu Leu Lys Val Val
               705                 710                 715
   tta gat tat gta gat aaa att tat aat gca gat att aaa cca gaa ata        2871
   Leu Asp Tyr Val Asp Lys Ile Tyr Asn Ala Asp Ile Lys Pro Glu Ile
           720                 725                 730
   aaa ata agt gcc aat caa tat gaa gtc aga ata aat aaa gat ggg aaa       2919
   Lys Ile Ser Ala Asn Gln Tyr Glu Val Arg Ile Asn Lys Asp Gly Lys
       735                 740                 745
   aaa gaa tta cta tct cat tca gga gag tgg ctt tct aaa gaa gaa gct       2967
   Lys Glu Leu Leu Ser His Ser Gly Glu Trp Leu Ser Lys Glu Glu Ala
   750                 755                 760                 765
   ata ata aaa gat att gcg agt aaa gaa att att tat ttt gat aca aga       3015
   Ile Ile Lys Asp Ile Ala Ser Lys Glu Ile Ile Tyr Phe Asp Thr Arg
                   770                 775                 780
   gaa aat act att aaa gca gaa gct aaa aat att atg gag ttg ata act       3063
   Glu Asn Thr Ile Lys Ala Glu Ala Lys Asn Ile Met Glu Leu Ile Thr
               785                 790                 795
   ttt aga aat agt cta gat aaa aaa tta aat gat tta gaa agt tta att       3111
   Phe Arg Asn Ser Leu Asp Lys Lys Leu Asn Asp Leu Glu Ser Leu Ile
           800                 805                 810
   aat aat aat ttt att gta aat gaa gta tat gaa caa tta aca tca aat       3159
   Asn Asn Asn Phe Ile Val Asn Glu Val Tyr Glu Gln Leu Thr Ser Asn
       815                 820                 825
   aat tta tat aga aca agt ttt aga aat tct ttt tta gat act ata aat       3207
   Asn Leu Tyr Arg Thr Ser Phe Arg Asn Ser Phe Leu Asp Thr Ile Asn
   830                 835                 840                 845
   ttt ata gaa gat gtt tct caa gag tta tat gaa ata aag ata aaa aat       3255
   Phe Ile Glu Asp Val Ser Gln Glu Leu Tyr Glu Ile Lys Ile Lys Asn
                   850                 855                 860
   aat ata aac gat gat tat atc atg gca tta gat gaa ata aag caa gat       3303
   Asn Ile Asn Asp Asp Tyr Ile Met Ala Leu Asp Glu Ile Lys Gln Asp
               865                 870                 875
   aat aat att tca gaa att aaa ttt att aat att aat tca gga gaa ttt       3351
   Asn Asn Ile Ser Glu Ile Lys Phe Ile Asn Ile Asn Ser Gly Glu Phe
           880                 885                 890
   agg gtt gta aga gaa aat agt agg tta att aat aaa ttt aaa gaa gat       3399
   Arg Val Val Arg Glu Asn Ser Arg Leu Ile Asn Lys Phe Lys Glu Asp
       895                 900                 905
   ttt aaa ttt atg att aat aat att aag agg ttg ata aaa ttt agt gat       3447
   Phe Lys Phe Met Ile Asn Asn Ile Lys Arg Leu Ile Lys Phe Ser Asp
   910                 915                 920                 925
   aat aac aag cta ata aca tca ttt gaa tta aag aat ata gag tca agc       3495
   Asn Asn Lys Leu Ile Thr Ser Phe Glu Leu Lys Asn Ile Glu Ser Ser
                   930                 935                 940
   agt gga gta agt aca tta aat aca agt ttt tta att caa tct atg att       3543
   Ser Gly Val Ser Thr Leu Asn Thr Ser Phe Leu Ile Gln Ser Met Ile
               945                 950                 955
   gat tat aaa gcg caa aac ttt gac ttc aat aaa tta agt aca tca gta       3591
   Asp Tyr Lys Ala Gln Asn Phe Asp Phe Asn Lys Leu Ser Thr Ser Val
           960                 965                 970
   aaa gta cag att tat tgt caa ata act aat ata agt tta tca gaa att        3639
   Lys Val Gln Ile Tyr Cys Gln Ile Thr Asn Ile Ser Leu Ser Glu Ile
       975                 980                 985
   caa gat gct agt aat tta gta aaa att ata gct  gaa gca aat gaa att       3687
   Gln Asp Ala Ser Asn Leu Val Lys Ile Ile Ala  Glu Ala Asn Glu Ile
   990                 995                 1000                1005
   gaa ata aat tta ata  cca aca tta gca aat  gct ata cca tta ata          3732
   Glu Ile Asn Leu Ile  Pro Thr Leu Ala Asn  Ala Ile Pro Leu Ile
                   1010                1015                1020
   aca aca ata gtt gat  gga ata aat ttg att  gca aat ata gat gaa          3777
   Thr Thr Ile Val Asp  Gly Ile Asn Leu Ile  Ala Asn Ile Asp Glu
                   1025                1030                1035
   tta ata aat act aaa  gat gaa tta cta aga  aag gaa tta gca gct          3822
   Leu Ile Asn Thr Lys  Asp Glu Leu Leu Arg  Lys Glu Leu Ala Ala
                   1040                1045                1050
   aga att gga att att  tca tct aat atg aca  gca gct att agt tct          3867
   Arg Ile Gly Ile Ile  Ser Ser Asn Met Thr  Ala Ala Ile Ser Ser
                   1055                1060                1065
   tat att tta tat ttt  act gaa ttt gga gaa  gta ttt aat cct tta          3912
   Tyr Ile Leu Tyr Phe  Thr Glu Phe Gly Glu  Val Phe Asn Pro Leu
                   1070                1075                1080
   tta gtg cca att gca  ggt ata tca agt ggt  att cct act tta gta          3957
   Leu Val Pro Ile Ala  Gly Ile Ser Ser Gly  Ile Pro Thr Leu Val
                   1085                1090                1095
   aat aat att tta att  tta gag gaa aaa tct  aaa gaa ata aca gaa          4002
   Asn Asn Ile Leu Ile  Leu Glu Glu Lys Ser  Lys Glu Ile Thr Glu
                   1100                1105                1110
   tat ttt tca cat ata  agt aaa gta gaa agt  gat gga tta ttt aaa          4047
   Tyr Phe Ser His Ile  Ser Lys Val Glu Ser  Asp Gly Leu Phe Lys
                   1115                1120                1125
   gtg tct gat aat aac  agt ata ttg att cca  ctt gat gat att gca          4092
   Val Ser Asp Asn Asn  Ser Ile Leu Ile Pro  Leu Asp Asp Ile Ala
                   1130                1135                1140
   att tct aag att gat  ttt aat aaa aga gaa  gtt atc tta gat aaa          4137
   Ile Ser Lys Ile Asp  Phe Asn Lys Arg Glu  Val Ile Leu Asp Lys
                   1145                1150                1155
   tta gat ata tgg gca  atg gaa gga gga tca  gga tta agt ggt aaa          4182
   Leu Asp Ile Trp Ala  Met Glu Gly Gly Ser  Gly Leu Ser Gly Lys
                   1160                1165                1170
   gaa act ttt ttt tct  gct ccg tat ata aat  gaa aat ctt cct aaa          4227
   Glu Thr Phe Phe Ser  Ala Pro Tyr Ile Asn  Glu Asn Leu Pro Lys
                   1175                1180                1185
   tta tct att gat aat  ttg ctt aag ata gat  ata aat aaa ata gat          4272
   Leu Ser Ile Asp Asn  Leu Leu Lys Ile Asp  Ile Asn Lys Ile Asp
                   1190                1195                1200
   ttt tca att aaa ggt  atg atg cta cct aat  ggc gtt agt aaa aca          4317
   Phe Ser Ile Lys Gly  Met Met Leu Pro Asn  Gly Val Ser Lys Thr
                   1205                1210                1215
   tta ggt tat gag ttc  aat aca gtt gcc aat  ata tat gaa tta gaa          4362
   Leu Gly Tyr Glu Phe  Asn Thr Val Ala Asn  Ile Tyr Glu Leu Glu
                   1220                 1225                 1230
   aat gat ggt gta aat  tta tta aat aga att  aga gat aat tat cca          4407
   Asn Asp Gly Val Asn  Leu Leu Asn Arg Ile  Arg Asp Asn Tyr Pro
                   1235                 1240                 1245
   gga cag ttt tat tgg  aga tat tat gct act  cta ttt aat tat ggt          4452
   Gly Gln Phe Tyr Trp  Arg Tyr Tyr Ala Thr  Leu Phe Asn Tyr Gly
                   1250                 1255                 1260
   atg gta aat tta aaa  att aat tat cat gac  aca gat gta aaa ata          4497
   Met Val Asn Leu Lys  Ile Asn Tyr His Asp  Thr Asp Val Lys Ile
                   1265                 1270                 1275
   aat tta gat aat gtt  gat cgt atg ttt ata  gta ccg aca tta aca          4542
   Asn Leu Asp Asn Val  Asp Arg Met Phe Ile  Val Pro Thr Leu Thr
                   1280                 1285                 1290
   ata gat caa gct aga  gaa aaa ctt tca tat  aat ttt aat gga gca          4587
   Ile Asp Gln Ala Arg  Glu Lys Leu Ser Tyr  Asn Phe Asn Gly Ala
                   1295                 1300                 1305
   gga gct aat tat tat  ata tac ctt tcg tct  caa cca ata aaa ata          4632
   Gly Ala Asn Tyr Tyr  Ile Tyr Leu Ser Ser  Gln Pro Ile Lys Ile
                   1310                 1315                 1320
   ttt att aat ggg act  aaa gaa gat aat tgg  att ctt aat ata gat          4677
   Phe Ile Asn Gly Thr  Lys Glu Asp Asn Trp  Ile Leu Asn Ile Asp
                   1325                 1330                 1335
   gat att gtt aaa gaa  gtt aca att gtt aat  aat agt ata gta aga          4722
   Asp Ile Val Lys Glu  Val Thr Ile Val Asn  Asn Ser Ile Val Arg
                   1340                 1345                 1350
   gga aag ttt att gaa  aat ata ttt aaa aaa  tta act att gag gat          4767
   Gly Lys Phe Ile Glu  Asn Ile Phe Lys Lys  Leu Thr Ile Glu Asp
                   1355                 1360                 1365
   aat aaa ata att ata  gga aat caa aaa ata  tat tta aaa aat aac          4812
   Asn Lys Ile Ile Ile  Gly Asn Gln Lys Ile  Tyr Leu Lys Asn Asn
                   1370                 1375                 1380
   aaa aca aat ata cat  ttt tct gta tct att  tta gat gga gtg aat          4857
   Lys Thr Asn Ile His  Phe Ser Val Ser Ile  Leu Asp Gly Val Asn
                   1385                 1390                 1395
   tta atg gta gaa gta  gac ttt aat aat aaa  atg tat cat tta tca          4902
   Leu Met Val Glu Val  Asp Phe Asn Asn Lys  Met Tyr His Leu Ser
                   1400                 1405                 1410
   ctt caa gga aac gaa  aaa aca ata tgt aat  aat atg aat att ata          4947
   Leu Gln Gly Asn Glu  Lys Thr Ile Cys Asn  Asn Met Asn Ile Ile
                   1415                 1420                 1425
   caa aat aac ata aat  aaa tta ttt ggc tct  tca aat ata aaa tca          4992
   Gln Asn Asn Ile Asn  Lys Leu Phe Gly Ser  Ser Asn Ile Lys Ser
                   1430                 1435                 1440
   ata cca tat ttc tat  aaa aat aat gga tta  gag aat tgt att gga          5037
   Ile Pro Tyr Phe Tyr  Lys Asn Asn Gly Leu  Glu Asn Cys Ile Gly
                       1445                1450                1455
   gta ttc tca ata aaa  aac aat gaa ttt att  tat atg tta aaa tat      5082
   Val Phe Ser Ile Lys  Asn Asn Glu Phe Ile  Tyr Met Leu Lys Tyr
                       1460                1465                1470
   gat gat aaa aat aaa  ata tat aaa tat aaa  gat aat aat tta gta      5127
   Asp Asp Lys Asn Lys  Ile Tyr Lys Tyr Lys  Asp Asn Asn Leu Val
                       1475                1480                1485
   tgt ggt ttt aat tta  ttt gat ata tct tat  gta gat ata att tct      5172
   Cys Gly Phe Asn Leu  Phe Asp Ile Ser Tyr  Val Asp Ile Ile Ser
                       1490                1495                1500
   aat gga gat aag aat  tat ttg aaa gga att  tgg aat aca tca gtt      5217
   Asn Gly Asp Lys Asn  Tyr Leu Lys Gly Ile  Trp Asn Thr Ser Val
                       1505                1510                1515
   aat aat tca gaa tat  aaa att gaa ttt tta  gtt gaa tta ata gac      5262
   Asn Asn Ser Glu Tyr  Lys Ile Glu Phe Leu  Val Glu Leu Ile Asp
                       1520                1525                1530
   caa aat act att gaa  atc att aaa ttg aat  tta agt gat gga tta      5307
   Gln Asn Thr Ile Glu  Ile Ile Lys Leu Asn  Leu Ser Asp Gly Leu
                       1535                1540                1545
   ata aaa tca ttt tta  act ata tta gat aat  tta gaa agt tta gat      5352
   Ile Lys Ser Phe Leu  Thr Ile Leu Asp Asn  Leu Glu Ser Leu Asp
                       1550                1555                1560
   gta aaa att tca aca  ata tat gat ttt tta  aaa aat att ctt ttt      5397
   Val Lys Ile Ser Thr  Ile Tyr Asp Phe Leu  Lys Asn Ile Leu Phe
                       1565                1570                1575
   agg gat gta ttt tta  gaa aat gag tca tta  aat ttt att gta agt      5442
   Arg Asp Val Phe Leu  Glu Asn Glu Ser Leu  Asn Phe Ile Val Ser
                       1580                1585                1590
   aaa ggc ttt gtt ttg  aca ggg aat aat tca  aaa aat aaa tac gag      5487
   Lys Gly Phe Val Leu  Thr Gly Asn Asn Ser  Lys Asn Lys Tyr Glu
                       1595                1600                1605
   ttt ata tgt aat agt  gaa aat ata caa tta  tat att act gaa ctt      5532
   Phe Ile Cys Asn Ser  Glu Asn Ile Gln Leu  Tyr Ile Thr Glu Leu
                       1610                1615                1620
   tac act aat ggt act  aaa ttt agg aat tgg  ctt gac aat ggt aag      5577
   Tyr Thr Asn Gly Thr  Lys Phe Arg Asn Trp  Leu Asp Asn Gly Lys
                       1625                1630                1635
   ata tta gta tca tca  aca ggt gaa agt tca  aat aat tta att gac      5622
   Ile Leu Val Ser Ser  Thr Gly Glu Ser Ser  Asn Asn Leu Ile Asp
                       1640                1645                1650
   ata taa                                                            5628
   Ile
<210> 17
   <211> 1651
   <212> PRT
   <213> Clostridium perfringens
<400> 17 201500092A2

## Description

The invention relates to a method of detecting avian necrotic enteritis, in which new markers which have been identified as suitable for detection of avian necrotic enteritis are used.

Clostridium perfringens is the main causative agent of avian necrotic enteritis (NE), an enteric disease of poultry that was first described in 1961. NE in chickens manifests as an acute or chronic enterotoxemia. The acute disease results in significant levels of mortality due to the development of necrotic lesions in the gut wall, whereas the chronic disease leads to a significant loss of productivity and welfare. It has been estimated that the disease results in damages of several billion US-Dollars per year for the poultry industry.

C. perfringens is commonly found in the gastrointestinal tract of poultry, the occurrence of necrotic enteritis, however, is sporadic. C. perfringens is a Gram-positive, rod-shaped, spore forming, oxygen-tolerant anaerobe. C. perfringes strains are classified into five toxin types (A, B, C, D and E), based on the production of four suspected major toxins (alpha, beta, epsilon and iota). While type A is consistently recovered from intestines of chicken, the other types are less common.

Early studies on NE suggested that the main virulence factor involved in the disease was secreted by the bacteria, which led to the proposal that a phospholipase C enzyme, called alpha-toxin, was the major toxin involved in pathogenesis. But recent studies showed that alpha-toxin seems not to be an essential virulence factor since alpha toxin mutant strains were capable of causing NE, which questions the role of alpha-toxin in the disease in general. In more recent studies, the novel pore forming toxin, netB, has been suggested to play a major key role in the development of this disease.

Apart from the mere association of the virulence factors per se to incidence of NE, there is still a great burden to show the mechanistic link between these factors to the establishment of the disease in birds or farm animals. Moreover, relating subclinical NE infection to any of these virulence factors is extremely difficult to achieve since birds are void of signs to warrant their examination earlier before slaughter. Therefore a need for an early method of detecting NE, and most importantly, the subclinical forms of NE remains imperative.

Traditionally, the discovery and validation of biomarkers for disease requires the use of tissue, either fresh frozen, or more commonly, formalin-fixed, paraffin-embedded. This has been especially true for mRNA based biomarkers, because RNase degradation of free RNA makes translation of tissue based biomarkers into biofluids such as blood or fecal/cecal samples highly problematic. But monitoring of tissue biomarkers is thus, because of its invasive nature, time-consuming and impractical, when large numbers of samples are involved like for farm animals.

Non-invasive methods such as the measurement of blood parameters have been described for the detection of diseases in birds (Chuku et al., 2012; Aade et al., 2012; Saleem, 2012). Gulbeena Saleem (2012) suggests as an alternative approach for the determination of sub-clinical NE the measurement of the levels of acute phase proteins like ceruplasmin, PIT54 and ovotransferrin in response to C. perfringes challenge in blood serum of the chickens. It turned out that only ceruplasmin seemed to be a suitable acute phase protein correlating with the occurrence of sub-clinical NE. Notwithstanding, this can be seen as a problem, since ceruplasmin is not a specific marker for NE but for diseases caused by other bacterial pathogens (collibacillosis in chicken, Piercy, 1979; salmonella infection in commercial layers, Garcia *et al.,* 2009), as well.

WO2008/148166 discloses a method relating to determining if an avian subject has been exposed to Clostridium perfringens by detecting a polynucleotide encoding NetB in a sample from the subject.

However, it was recently discovered that exosomes and other membrane vesicles found in biomaterials contain stable RNA from human donor cells, including mRNA, miRNA and other non-coding RNA with high specificity to disease conditions (Skog et al., 2008; Nilsson et al., 2009; Li et al., 2009; Shao et al., 2012).

Thus the inventors of the current application wondered, if membrane vesicles isolated from bodily fluids or excrements of poultry might be used to determine poultry exhibiting sub-clinical necrotic enteritis. This might be in particular true due to an accumulation of specific host miRNAs showing correlation to the disease.

Surprisingly it was found that microvesicles isolated from avian bodily fluids or avian excrements did exhibit an alteration in the content of detectable RNA in dependence of the presence of necrotic enteritis. Besides an increase of specific host-miRNAs in the microvesicles due to infection by C. perfringens, surprisingly also mRNA sequences of C. perfringens could be detected in the microvesicle fraction, which are therefore classified as disease specific virulence factors and accordingly as suitable biomarkers indicative for necrotic enteritis.

The occurrence of C. perfringens mRNA in the microvesicle fraction might be explained by the fact that the isolation procedure applied does not only lead to an enrichment of microvesicles of poultry, but also to an enrichment of microvesicles of the infectious bacterium. Surprisingly, besides known putative virulence factors like netB, also previously non-suspective RNA sequences could be identified as being correlative to necrotic enteritis.

It was thus a first object of the current invention to provide a fast and reliable, preferably non-invasive, method for determining avian necrotic enterititis, in particular sub-clinical avian necrotic enterititis, preferably in poultry, more preferably in chicken and/or to identify avian subjects which suffer from necrotic enteritis.

It was a further object of the current invention to identify suitable markers, preferably disease-specific markers, which can be employed in such a method.

The invention is defined by the scope of the appended claims.

The markers indicative for necrotic enteritis as identified according to the invention are selected from the following sequences of Clostridium perfringens and homologues and fragments thereof: the SAM region (SEQ ID NO: 1), the swim zinc finger region (SEQ ID NO: 3), the scRNA (SEQ ID NO: 4), the colA region (SEQ ID NO: 5), the CPE0956 region (SEQ ID NO: 7, comprising the VR.RNA sequence) and the virX region (SEQ ID NO: 9); as well as from the following avian sequences and homologues and fragments thereof: mir-16 (SEQ ID NO: 10), mir-24 (SEQ ID NO: 11), mir-155 (SEQ ID NO: 12), mir-206 (SEQ ID NO: 13).

As it is surprising that the SAM region (SEQ ID NO: 1), the swim zinc finger region (SEQ ID NO: 3), the scRNA (SEQ ID NO: 4), the CPE0956 region (SEQ ID NO: 7, comprising the VR.RNA sequence), the virX region (SEQ ID NO: 9) as well as mir-16 (SEQ ID NO: 10), mir-24 (SEQ ID NO: 11), mir-155 (SEQ ID NO: 12), and mir-206 (SEQ ID NO: 13) can be used as markers for determining avian necrotic enteritis, at all, thus subject of the invention is a method of detecting avian necrotic enteritis, in particular sub-clinical avian necrotic enteritis, wherein detection of necrotic enteritis is carried out by detecting the amount of at least one of the following sequences in an avian sample, preferably in microvesicles of an avian sample, in particular in comparison to a control sample of a non-infected avian, wherein an increased level of at least one of those sequences in comparison to a non-infected control is indicative for necrotic enteritis:
a) polynucleotides which have a sequence identity of at least 95 %, preferably 100 %, to a polynucleotide which consists of at least 40, preferably at least 60, 100, 150, 180 or 200, consecutive nucleotides of the nucleotide sequence as depicted in SEQ ID NO: 1,
b) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 3,
c) polynucleotides which have a sequence identity of at least 95 %, preferably 100 %, to a polynucleotide which comprises at least 20,
   preferably at least 25, 40, 60, 100, 150, 180 or 200, consecutive nucleotides of the nucleotide sequence as depicted in SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 7;
e) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 9;
f) polynucleotides which have a sequence identity of at least 95 %, preferably 100 %, to a polynucleotide which consists of at least 8 or 10, preferably at least 12, 14, 16, 18, 20, 25, 40, 60 or 80, consecutive nucleotides of the nucleotide sequence as depicted in SEQ ID NO: 10;
g) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 11;
h) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 12;
i) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 13;
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

In this method the sequences are preferably selected from the following group:
a) polynucleotides which have a sequence identity of at least 95 %, preferably 100 %, to a polynucleotide which consists of at least 40, preferably at least 60, 100, 150, 180 or 200 consecutive nucleotides of the nucleotide sequence according to position 1 to 630, in particular 420 to 630, preferably 460 to 560, of SEQ ID NO: 1,
b) polynucleotides which have a sequence identity of at least 95 %, preferably 100 %, to a polynucleotide which consists of at least 20,
   preferably at least 25, 40, 60, 100, 150, 180 or 200, consecutive nucleotides of the nucleotide sequence according to position 600 to 1000, in particular 670 to 760, of SEQ ID NO: 3,
c) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence according to position 60 to 240, in particular 80 to 220, of SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence according to position 1 to 591, in particular 100 to 280 of SEQ ID NO: 7;
e) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence according to position 12 to 418, in particular 240 to 400 of SEQ ID NO: 9;
f) polynucleotides which have a sequence identity of at least 95 %, preferably 100 %, to a polynucleotide which consists of at least 8 or 10, preferably at least 12, consecutive nucleotides of the nucleotide sequence according to position 14 to 25 of SEQ ID NO: 10;
g) polynucleotides which have a sequence identity of at least 95 %, preferably 100 %, to a polynucleotide which which consists of at least 8 or 10, preferably at least 12, 14, 16, 18 or 20, consecutive nucleotides of the nucleotide sequence according to position 44 to 65 of SEQ ID NO: 11;
h) polynucleotides which have a sequence identity of at least 95 %, preferably 100 %, to a polynucleotide which consists of at least 8 or 10, preferably at least 12, 14, 16, 18 or 20, consecutive nucleotides of the nucleotide sequence according to position 3 to 24 of SEQ ID NO: 12;
i) polynucleotides which have a sequence identity of at least 95 %, preferably 100 %, to a polynucleotide which consists of at least 8 or 10, preferably at least 12, 14, 16, 18 or 20, consecutive nucleotides of the nucleotide sequence according to position 45 to 66 of SEQ ID NO: 13,
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

More preferably the sequences are selected from the following group:
a) polynucleotides which have a sequence identity of at least 95 %, preferably at least 98 or 99 %, most preferably 100 %, to a polynucleotide which consists of at least 40 consecutive nucleotides of the nucleotide sequence according to position 460 to 560 of SEQ ID NO: 1,
b) polynucleotides which have a sequence identity of at least 95 %, preferably at least 98 or 99 %, most preferably 100 %, to a polynucleotide which consists of at least 20, preferably at least 30 or 40, consecutive nucleotides of the nucleotide sequence according to position 670 to 760 of SEQ ID NO: 3,
c) polynucleotides which have a sequence identity of at least 95 %, preferably at least 98 or 99 %, most preferably 100 %, to a polynucleotide which consists of at least 20, preferably at least 30 or 40, consecutive nucleotides of the nucleotide sequence according to position 80 to 220 of SEQ ID NO: 4;
d) polynucleotides which have a sequence identity of at least 95 %, preferably at least 98 or 99 %, most preferably 100 %, to a polynucleotide which consists of at least 20, preferably at least 30 or 40, consecutive nucleotides of the nucleotide sequence according to position 100 to 280 of SEQ ID NO: 7;
e) polynucleotides which have a sequence identity of at least 95 %, preferably at least 98 or 99 %, most preferably 100 %, to a polynucleotide which consists of at least 20, preferably at least 30 or 40, consecutive nucleotides of the nucleotide sequence according to position 240 to 400 of SEQ ID NO: 9;
f) polynucleotides which have a sequence identity of at least 95 %, preferably at least 98 or 99 %, most preferably 100 %, to a polynucleotide which consists of at least 8 or 10, preferably at least 12, consecutive nucleotides of the nucleotide sequence according to position 14 to 25 of SEQ ID NO: 10;
g) polynucleotides which have a sequence identity of at least 95 %, preferably at least 98 or 99 %, most preferably 100 %, to a polynucleotide which which consists of at least 8 or 10, preferably at least 12, 14, 16, 18 or 20, consecutive nucleotides of the nucleotide sequence according to position 44 to 65 of SEQ ID NO: 11;
h) polynucleotides which have a sequence identity of at least 95 %, preferably at least 98 or 99 %, most preferably 100 %, to a polynucleotide which consists of at least 8 or 10, preferably at least 12, 14, 16, 18 or 20, consecutive nucleotides of the nucleotide sequence according to position 3 to 24 of SEQ ID NO: 12;
i) polynucleotides which have a sequence identity of at least 95 %, preferably at least 98 or 99 %, most preferably 100 %, to a polynucleotide which consists of at least 8 or 10, preferably at least 12, 14, 16, 18 or 20, consecutive nucleotides of the nucleotide sequence according to position 45 to 66 of SEQ ID NO: 13,
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

In a preferred embodiment of the invention combinations of said polynucleotides, preferably combinations of at least 2, 3, 4, 5, 6 or 7 of said polynucleotides are used for detecting necrotic enteritis.

The avian sample according to the invention can be a tissue sample, preferably an intestinal sample, in particular a duodenal or a jejunal sample.

Preferably the avian sample is selected from avian bodily fluids and avian excrements and solutions and suspensions thereof.

The avian bodily fluid according to the invention is preferably blood, blood serum or blood plasma.

The avian excrement according to the invention is preferably selected from fecal and cecal excrements.

Suitable sample volumes are, for example, 0.1 to 20 ml, in particular 0.2 to 10 ml, preferably 0.5 to 5 ml. Suitable sample masses are, for example 0.1 to 20 g, in particular 0.2 to 10 g, preferably 0.5 to 5 g.

The avian subject according to the invention is preferably poultry.

Preferred poultry according to the invention are chickens, turkeys, ducks and geese. The poultry can be optimized for producing young stock. This type of poultry is also referred to as parent animals. Preferred parent animals are, accordingly, parent broilers, parent ducks, parent turkeys and parent geese.

The poultry according to the invention can also be selected from fancy poultry and wild fowl.

Preferred fancy poultry or wild fowl are peacocks, pheasants, partridges, guinea fowl, quails, capercaillies, grouse, pigeons and swans.

Further preferred poultry according to the invention are ostriches and parrots.

Most preferred poultry according to the invention are chickens.

As used herein, the term "polynucleotides" or "nucleic acids" refer to DNA and RNA. The polynucleotides and nucleic acids can be single stranded or double stranded.

It turned out that the occurrence of markers indicative for necrotic enteritis is sample-specific, i.e. the detectable amount of the different markers is dependent on the sample type.

So it was found that in fecal excrements the best marker is the SAM region, followed by scRNA, virX region, colA region, swim zinc finger region and the CPE0956 region, including the VR.RNA sequence.

To the contrary, in cecal excrements the best marker is also the SAM region, but is followed by the swim zinc finger region, scRNA, mir206, the virX region, the CPE0956 region, including the VR.RNA sequence, and the colA region.

Further in blood samples the best marker is mir16, followed by mir155 and mir24.

Thus a preferred embodiment of the application is a method of detecting avian necrotic enteritis, in particular sub-clinical avian necrotic enteritis, the method comprising determining the level of at least one marker indicative for necrotic enteritis in avian fecal excrements, preferably in microvesicles isolated from avian fecal excrements, wherein an increased level of this at least one marker in comparison to a non-infected control is indicative for necrotic enteritis and wherein the marker is selected from
a) polynucleotides which have a sequence identity of at least 95 %, preferably 100 %, to a polynucleotide which consists of at least 40, preferably at least 60, 100, 150, 180 or 200, consecutive nucleotides of the nucleotide sequence as depicted in SEQ ID NO: 1,
b) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 3,
c) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 5;
e) polynucleotides which have a sequence identity of at least 95 %, preferably 100 %, to a polynucleotide which consists of at least 20,
   preferably at least 25, 40, 60, 100, 150, 180 or 200, consecutive nucleotides of the nucleotide sequence as depicted in SEQ ID NO: 7;
f) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 9;
g) polynucleotides (DNAs and RNAs) which are complementary to the sequences according to (a) to (f);
h) polynucleotides (DNAs and RNAs) which are complementary to the sequences according to (g);
i) polynucleotides which comprise the polynucleotides according to (a) to (h).

In this method the sequences are preferably selected from the following group:
a) polynucleotides which have a sequence identity of at least 95 %, preferably 100 %, to a polynucleotide which consists of at least 40, preferably at least 60 or 100, consecutive nucleotides of the nucleotide sequence according to position 1 to 630, in particular 420 to 630, preferably 460 to 560, of SEQ ID NO: 1,
b) polynucleotides which have a sequence identity of at least 95 %, preferably 100 %, to a polynucleotide which consists of at least 20, preferably at least 25, 40, 60 or 90, consecutive nucleotides of the nucleotide sequence according to position 600 to 1000, in particular 670 to 760, of SEQ ID NO: 3,
c) polynucleotides which have a sequence identity of at least 95 %, preferably 100 %, to a polynucleotide which consists of at least 20, preferably at least 25, 40, 60, 100 or 140, consecutive nucleotides of the nucleotide sequence according to position 60 to 240, in particular 80 to 220, of SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides according to position 1300 to 1800, in particular 1400 to 1700, of SEQ ID NO: 5;
e) polynucleotides which have a sequence identity of at least 95 %, preferably 100 %, to a polynucleotide which consists of at least 20, preferably at least 25, 40, 60, 100, 150 or 180, consecutive nucleotides of the nucleotide sequence according to position 1 to 591, in particular 100 to 280 of SEQ ID NO: 7;
f) polynucleotides which have a sequence identity of at least 95 %, preferably 100 %, to a polynucleotide which consists of at least 20, preferably at least 25, 40, 60, 100, 150 or 160, consecutive nucleotides of the nucleotide sequence according to position 12 to 418, in particular 240 to 400 of SEQ ID NO: 9;
g) polynucleotides (DNAs and RNAs) which are complementary to the sequences according to (a) to (f);
h) polynucleotides (DNAs and RNAs) which are complementary to the sequences according to (g);
i) polynucleotides which comprise the polynucleotides according to (a) to (h).

Thus another preferred embodiment of the application is a method of detecting avian necrotic enteritis, in particular sub-clinical avian necrotic enteritis, the method comprising determining the level of at least one marker indicative for necrotic enteritis in avian cecal excrements, in particular in microvesicles isolated from avian cecal excrements, wherein an increased level of this at least one marker in comparison to a non-infected control is indicative for necrotic enteritis and wherein the marker is selected from
a) polynucleotides which have a sequence identity of at least 95 %, preferably 100 %, to a polynucleotide which consists of at least 40, preferably at least 60, 100, 150, 180 or 200, consecutive nucleotides of the nucleotide sequence as depicted in SEQ ID NO: 1,
b) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 3,
c) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 5;
e) polynucleotides which have a sequence identity of at least 95 %, preferably 100 %, to a polynucleotide which consists of at least 20,
   preferably at least 25, 40, 60, 100, 150, 180 or 200, consecutive nucleotides of the nucleotide sequence as depicted in SEQ ID NO: 7;
f) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 9;
g) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 13;
h) polynucleotides (DNAs and RNAs) which are complementary to the sequences according to (a) to (g);
i) polynucleotides (DNAs and RNAs) which are complementary to the sequences according to (h);
j) polynucleotides which comprise the polynucleotides according to (a) to (i).

In this method the sequences are preferably selected from the following group:
a) polynucleotides which have a sequence identity of at least 95 %, preferably 100 %, to a polynucleotide which consists of at least 40, preferably at least 60 or 100, consecutive nucleotides of the nucleotide sequence according to position 1 to 630, in particular 420 to 630, preferably 460 to 560, of SEQ ID NO: 1,
b) polynucleotides which have a sequence identity of at least 95 %, preferably 100 %, to a polynucleotide which consists of at least 20, preferably at least 25, 40, 60 or 90, consecutive nucleotides of the nucleotide sequence according to position 600 to 1000, in particular 670 to 760, of SEQ ID NO: 3,
c) polynucleotides which have a sequence identity of at least 95 %, preferably 100 %, to a polynucleotide which consists of at least 20, preferably at least 25, 40, 60, 100 or 140, consecutive nucleotides of the nucleotide sequence according to position 60 to 240, in particular 80 to 220, of SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides according to position 1300 to 1800, in particular 1400 to 1700, of SEQ ID NO: 5;
e) polynucleotides which have a sequence identity of at least 95 %, preferably 100 %, to a polynucleotide which consists of at least 20,
   preferably at least 25, 40, 60, 100, 150 or 180, consecutive nucleotides of the nucleotide sequence according to position 1 to 591, in particular 100 to 280 of SEQ ID NO: 7;
f) polynucleotides which have a sequence identity of at least 95 %, preferably 100 %, to a polynucleotide which consists of at least 20, preferably at least 25, 40, 60, 100, 150 or 160, consecutive nucleotides of the nucleotide sequence according to position 12 to 418, in particular 240 to 400 of SEQ ID NO: 9;
g) polynucleotides which have a sequence identity of at least 95 %, preferably 100 %, to a polynucleotide which consists of at least 8 or 10, preferably at least 12, 14, 16, 18 or 20, consecutive nucleotides of the nucleotide sequence according to position 45 to 66 of SEQ ID NO: 13,
h) polynucleotides (DNAs and RNAs) which are complementary to the sequences according to (a) to (g);
i) polynucleotides (DNAs and RNAs) which are complementary to the sequences according to (h);
j) polynucleotides which comprise the polynucleotides according to (a) to (i).

Thus another preferred embodiment of the application is a method of detecting avian necrotic enteritis, in particular sub-clinical necrotic enteritis, the method comprising determining the level of at least one marker indicative for necrotic enteritis in avian blood, in particular in microvesicles isolated from avian blood, wherein an increased level of this at least one marker in comparison to a non-infected control is indicative for necrotic enteritis and wherein the at least one marker is selected from
a) polynucleotides which have a sequence identity of at least 95 %, preferably 100 %, to a polynucleotide which consists of at least 8 or 10, preferably at least 12, 14, 16, 18, 20, 25, 40, 60 or 80, consecutive nucleotides of the nucleotide sequence as depicted in SEQ ID NO: 10;
b) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 11;
c) polynucleotides which have a sequence identity of at least 95 %, preferably 100 %, to a polynucleotide which consists of at least 8 or
   10, preferably at least 12, 14, 16, 18, 20, 25, 40 or 60, consecutive nucleotides of the nucleotide sequence as depicted in SEQ ID NO: 12;
d) polynucleotides (DNAs and RNAs) which are complementary to the sequences according to (a) to (c);
e) polynucleotides (DNAs and RNAs) which are complementary to the sequences according to (d);
f) polynucleotides which comprise the polynucleotides according to (a) to (e).

In this method the sequences are preferably selected from the following group:
a) polynucleotides which have a sequence identity of at least 95 %, preferably 100 %, to a polynucleotide which consists of at least 8 or 10, preferably at least 12, consecutive nucleotides of the nucleotide sequence according to position 14 to 25 of SEQ ID NO: 10;
b) polynucleotides which have a sequence identity of at least 95 %, preferably 100 %, to a polynucleotide which which consists of at least 8 or 10, preferably at least 12, 14, 16, 18 or 20, consecutive nucleotides of the nucleotide sequence according to position 44 to 65 of SEQ ID NO: 11;
c) polynucleotides which have a sequence identity of at least 95 %, preferably 100 %, to a polynucleotide which consists of at least 8 or 10, preferably at least 12, 14, 16, 18 or 20, consecutive nucleotides of the nucleotide sequence according to position 3 to 24 of SEQ ID NO: 12;
d) polynucleotides (DNAs and RNAs) which are complementary to the sequences according to (a) to (c);
e) polynucleotides (DNAs and RNAs) which are complementary to the sequences according to (d);
f) polynucleotides which comprise the polynucleotides according to (a) to (e).

ColA stands for Collagenese A of Clostridium perfringens; this gene might be associated with the pathogenesis of NE because of its similarity to the so called kappa-toxin, one of the supposed major toxins of this bacterium (Obana et al., 2013).

Nevertheless, the occurrence as well as the deregulation of these genes in microvesicles could not have been expected.

The virX gene is known to regulate the plx, colA, and pfoA genes in C. perfringens (Ohtani et al., 2002), but its involvement in the pathogenesis of NE has not been reported before.

The small cytoplasmic RNA (scRNA), a member of an evolutionarily conserved signal-recognition-particle-like RNA family involved in the sporulation mechanism of Clostridium perfringens (Nakamura et al., 1995) has also not been reported previously to be in direct association with NE in chickens.

Furthermore, the involvement of the SAM domain, the swim zinc finger domain and the CPE0956 region, including the VR.RNA, in the pathogenesis of necrotic enteritis has not been disclosed before and could also not have been expected as these sequences are sequences with previously unknown functions.

The eukaryotic version of membrane vesicles is also called exosomes. Exosomes have typically a diameter of 30-200 nm. They are shed into all biofluids and carry many intact proteins and oligonucleotides, as they are protected from degredation by RNAses (Koga et al., 2011) Exosomes have been studied in particular in cancer and immune cells.

Microvesicles from bacteria are also called extracellular membrane vesicles (MVs) or outer membrane vesicles (OMVs). They are spherical bilayer structures with average diameter of typically 20-500 nm. Bacterial microvesicles have first been described for Gram-negative bacteria, but later also for some Gram-positive bacteria. In a recent study, it was reported that microvesicles are produced and released also by C. perfringens type A strains, triggering innate and adaptive immune responses. It was further found out in the course of this study that important virulence factors like alpha-toxin and NetB were not present in the membrane vesicles, whereas beta2-toxin was found in the membrane vesicles (Jiang et al., (2014).

MicroRNAs (miRNAs) are small, non-coding RNAs that are usually 18-23 nucleotides in size and have been suggested to play a critical role in the regulation of gene expression. In particular the genes associated with immune responses have been reported to be highly targeted by miRNAs. In chickens miRNA expression has been studied in embryo developmental processes, germ cell development, immune organs and diseases.

In recent studies the miRNA profile of two different highly inbred White Leghorn chicken lines infected by C. perfringens was investigated (Dinh et al., 2014; Hong et al., 2014). RNA analysis was carried out with tissue from spleen and intestinal mucosa. The results suggest that some miRNAs are differentially altered in response to necrotic enteritis and that they modulate the expression of their target genes. Microvesicles were not investigated in these studies.

The miRNAs as found to be correlative to necrotic enteritis according to the current invention were investigated before by Wang et al. in connection with avian influenza virus infected broilers (Wang et al., 2012). Wang et al. found that the miRNAs miR-155, miR-16-1 and miR-24 are expressed stronger in the lungs of infected broilers than in those of non-infected ones, but they found further that the miR-206 is, to the contrary, stronger expressed in non-infected ones than in infected ones. Thus the use of miR-206 as marker for a bacterial infection is disclosed in the current application for the first time. Further in contrast to Wang et al. according to the current invention all miRNAs were detected in microvesicles, not in tissue.

Applications of the methods according to the invention are for example (i) aiding in the diagnosis and/or prognosis of avian necrotic enteritis, (ii) monitoring the progress or reoccurrence of avian necrotic enteritis, or (iii) aiding in the evaluation of treatment efficacy for an avian subject undergoing or contemplating treatment for necrotic enteritis; wherein the presence or absence of one or more of the biomarkers mentioned before in the nucleic acid extraction obtained from the method is determined, and the one or more biomarkers are associated with the diagnosis, progress or reoccurrence, or treatment efficacy, respectively, of necrotic enteritis.

Applications of the invention in particular help to avoid loss in avian performance like weight gain and feed conversion.

### Isolation of microvesicles

The isolation of microvesicles from a biological sample prior to extraction of nucleic acids is advantageous, because nucleic acid-containing microvesicles produce significantly higher yields of nucleic acid species with higher integrity as compared to the yield/integrity obtained by extracting nucleic acids directly from the fluid sample or excrement without first isolating microvesicles. Further it is also possible to detect nucleic acids which are expressed at low levels. The isolation of microvesicles also allows the exclusion of proteins, lipids, cell debris, cells and other potential contaminants and PCR inhibitors that are naturally found within biological samples.

Isolation of membrane vesicles from biological samples and subsequent extraction of the nucleic acids can be done for example by ultracentrifugation, e.g., spinning at more than 10,000 g for 1-3 hours, followed by removal of the supernatant, washing the pellet, lysing the pellet and purifying the nucleic acids on a column. Alternative methods are ultrafiltration, e.g., using 100 kD filters, polymer precipitation techniques, and/or filtration based on size.

Further alternative methods are differential centrifugation as described by Raposo et al (1996), Skog et. al(2008) and Nilsson et. al (2009); ion exchange and/or gel permeation chromatography as described in US Patent Nos. 6,899,863 and 6,812,023; sucrose density gradients or organelle electrophoresis as described in U.S. Patent No. 7,198,923; magnetic activated cell sorting (MACS) as described by Taylor and Gercel-Taylor (2008); nanomembrane ultrafiltration concentration as described by Cheruvanky et al(2007); Percoll gradient isolation as described by Miranda et al ( 2010); and, isolation by a microfluidic device Chen et al(2010).

Capture surface based isolation of microvesicles according to WO 2014/107571
In a preferred embodiment the microvesicle fraction is bound to a capture surface, in particular to a membrane, a filter or to a surface modified bead as disclosed in WO 2014/107571.

The biological sample is preferably dissolved in a loading buffer, which has preferably a pH of 4-8, more preferably 5-7. Thus, binding to the capture surface takes preferably place at a pH of 4-8, more preferably 5-7. Preferably, after contacting the biological sample with the capture surface, one or more wash steps are performed.

The loading and wash buffers which can be employed according to the invention can be of high or low ionic strength. Thus, the salt concentration, e.g., NaCl concentration, can be from 0 to 2.4M. Preferably, the buffers include one or more of the following components: Tris, Bis-Tris, Bis-Tris-Propane, Imidazole, Citrate, Methyl Malonic Acid, Acetic Acid, Ethanolamine, Diethanolamine, Triethanolamine (TEA) and Sodium phosphate.

Washing preferably takes place by using wash buffer comprising 250 mM Bis Tris Propane and exhibiting a pH of 6.5-7.0.

By adding detergents to the wash buffer nonspecific binding is suppressed. Detergents suitable for use include, but are not limited to, sodium dodecyl sulfate (SDS), Tween-20, Tween-80, Triton X-100, Nonidet P-40 (NP-40), Brij-35, Brij-58, octyl glucoside, octyl thioglucoside, CHAPS or CHAPSO.

After washing of the capture surface the nucleic acid can be released by lysing the fixed microvesicles. For lysing the fixed microvesicles preferably a phenol-based lysis buffer is used, like QIAzol® lysis reagent (Qiagen, Germany). Purification of the nucleic acid can then be done by chloroform extraction using PLG tubes, followed by ethanol conditioning. The nucleic acid can then be bound to a silica column before washing and eluting.

The lysis of microvesicles and extraction of nucleic acids may also be achieved by using a commercially available Qiagen R easy Plus kit, a commercially available Qiagen miR easy kit or by using phenolchloroform according to standard procedures and techniques known in the art. Such methods may also utilize a nucleic acid-binding column to capture the nucleic acids contained within the microvesicles. Once bound, the nucleic acids can then be eluted using a buffer or solution suitable to disrupt the interaction between the nucleic acids and the binding column.

The nucleic acid extraction methods may also include the use of further agents like an RNase inhibitor such as Superase-In (commercially available from Ambion Inc.) or RNaseINplus (commercially available from Promega Corp.); a protease (which may also function as an RNase inhibitor); DNase; a reducing agent; a decoy substrate such as a synthetic RNA and/or carrier RNA; a soluble receptor that can bind RNase; a small interfering RNA (siRNA); an RNA binding molecule, such as an anti-RNA antibody, a basic protein or a chaperone protein; an RNase denaturing substance, such as a high osmolarity solution, a detergent, or a combination thereof. The RNAse inhibitors as well as the further agents may be added to the biological sample, and/or to the isolated microvesicle fraction, prior to extracting nucleic acids.

These further agents may exert their functions in various ways, e.g., through inhibiting RNase activity (e.g., RNase inhibitors), through a ubiquitous degradation of proteins (e.g., proteases), or through a chaperone protein (e.g., a RNA-binding protein) that binds and protects RNAs. In all instances, such extraction enhancement agents remove or at least mitigate some or all of the adverse factors in the biological sample or associated with the isolated particles that would otherwise prevent or interfere with the high quality extraction of nucleic acids from the isolated particles.

The quantification of 18S and 28S rRNAs extracted can be used to determine the quality of the nucleic acid extraction.

The capture surface can be neutral, negatively charged or a positively charged. That means, in principle any kind of capture surface can be used. Depending on the membrane material, the pore sizes of the membrane can range from 20 nm to 5 µm.

In particular the capture surface can be a commercially available membrane like Mustang® Ion Exchange Membrane from PALL Corporation; Vivapure ® Q, Vivapure® Q Maxi H; Sartobind ® D, Sartobind (S), Sartobind ® Q, Sartobind ® IDA or Sartobind® Aldehyde from Sartorius AG; Whatman® DE81 from Sigma; Fast Trap Virus Purification column from EMD Millipore; or Thermo Scientific* Pierce Strong Cation and Anion Exchange Spin Columns.

When a membrane is used for fixing the nucleic acids, then the membrane can be made from a variety of suitable materials, for example it can be a polyethersulfone (PES) (e.g., from Millipore or PALL Corp.) or regenerated cellulose (RC) (e.g., from Sartorius or Pierce).

When a negatively charged membrane is used, then the capture surface is preferably an S membrane, which is a negatively charged membrane and is a cation exchanger with sulfonic acid groups. Preferably, the S membrane is functionalized with sulfonic acid, R-CH₂-SO₃". Alternatively, the negatively charged capture surface is a D membrane, which is a weak basic anion exchanger with diethylamine groups, R-CH₂-NFT(C₂H₅)₂. Alternatively, the capture surface is a metal chelate membrane. For example, the membrane is an IDA membrane, functionalized with minodiacetic acid -N(CH₂COOH")₂. In some embodiments, the capture surface is a microporous membrane, functionalized with aldehyde groups, -CHO. In other embodiments, the membrane is a weak basic anion exchanger, with diethylamino ethyl (DEAE) cellulose.

When a neutral capture surface is used, then the capture surface is preferably a filter selected from the group consisting of 20nm neutral PES syringe filtration (Tisch and Exomir), 30nm neutral PES syringe filtration (Sterlitech), 50nm neutral PES syringe filtration (Sterlitech), 0.2um neutral PES homemade spin column filtration (Pall), 0.8um neutral PES homemade spin column filtration (Pall) and 0.8um neutral PES syringe filtration (Pall). In embodiments where the capture surface is neutral, preferably the neutral capture surface is not housed in a syringe filter.

The charged surface is preferably selected from the group consisting of 0.65um positively charged Q PES vacuum filtration, 3-5um positively charged Q RC spin column filtration, 0.8um positively charged Q PES homemade spin column filtration, 0.8um positively charged Q PES syringe filtration, 0.8um negatively charged S PES homemade spin column filtration, 0.8um negatively charged S PES syringe filtration, and 50nm negatively charged nylon syringe filtration.

In the preferred embodiment, when the surface is a positively charged membrane, a Q membrane, which is a positively charged membrane and is an anion exchanger with quaternary amines, can be used. In a preferred embodiment the Q membrane is functionalized with quaternary ammonium, R-CH₂-N⁺(CH₃)₃.

In a very preferred embodiment the positively charged membrane is a regenerated cellulose, strong basic anion exchanger ("RC/SBAE") membrane, which is an anion exchanger with quaternary amines. In a preferred embodiment the RC/SBAE membrane is functionalized with quaternary ammonium, R-CH₂-N⁺(CH₃)₃. The pore size of the membrane is preferably at least 3 µm.

The capture surface, e.g., membrane, can be housed within a device used for centrifugation; e.g. spin columns, or for vacuum application e.g. vacuum filter holders, or for filtration with pressure e.g. syringe filters.

In a spin-column based purification process preferably more than 1 membrane, preferably 2 to 5 membranes, are used in parallel in a tube, wherein the membranes are preferably all directly adjacent to one another at one end of the column. The collection tube may be commercially available, like a 50ml Falcon tube. The column is preferably suitable for spinning, i.e., the size is compatible with standard centrifuge and micro-centrifuge machines.

Instead of a membrane also a bead with the same surface modification can be used for capturing the nucleic acid. If a bead is used, it can be magnetic or non-magnetic.

If blood is used as a sample, blood can for example be collected into K2 EDTA tubes and mixed well with complete inversions. Then the tubes are centrifuged at 1300xg for 10 min to separate the blood cells and plasma. Then plasma is removed and filtered through a 0.8µm filter to remove cell debris and platelets. All plasma samples are then aliquoted into 1ml cryovials and stored at -80°C until use.

Before RNA isolation, the membrane is preferably conditioned by passing through equilibrium buffer. The thawed plasma sample is diluted with loading buffer. The diluted plasma sample is slowly passed through the membrane that adsorbs the microvesicles. The membrane is then washed with a wash buffer to remove any weakly bound plasma components. Then a lysis reagent is passed through the membrane to lyse the microvesicles. RNA is isolated using the miRNeasy kit (Qiagen).

RNA can be assessed for quality and concentration with the 2100 Bioanalyzer (Agilent) using a RNA 6000 Pico Chip. The relative quantity of the extracted RNA is measured by RT-qPCR using selected human gene expression assays from Applied Biosystems (Taqman Assay).

In preferred embodiments, in particular when solubilized fecal or cecal samples are used, a pre-processing step prior to isolation, purification or enrichment of the microvesicles is performed to remove large unwanted particles, cells and/or cell debris and other contaminants present in the biological sample. The preprocessing steps may be achieved through one or more centrifugation steps (e.g., differential centrifugation) or one or more filtration steps (e.g., ultrafiltration), or a combination thereof. Where more than one centrifugation pre-processing steps are performed, the biological sample may be centrifuged first at a lower speed and then at a higher speed. If desired, further suitable centrifugation pre-processing steps may be carried out. Alternatively or in addition to the one or more centrifugation pre-processing steps, the biological sample may be filtered. For example, a biological sample may be first centrifuged at a low speed of about 100-500g, preferably 250-300g, and after that at a higher speed of about 2,000 to about 200,000 g, preferably at about 20,000g, for 10 minutes to about 1 hour, preferably at a temperature of 0-10°C, to remove large unwanted particles; the sample can then be filtered, for example, by using a filter with an exclusion size of 0.1 to 1.0 µm.

The microvesicles in the pellet obtained by centrifugation at higher speeds may be reconstituted with a smaller volume of a suitable buffer for the subsequent steps of the process. The concentration step may also be performed by ultrafiltration. In fact, this ultrafiltration both concentrates the biological sample and performs an additional purification of the microvesicle fraction. In another embodiment, the filtration is an ultrafiltration, preferably a tangential ultrafiltration. Tangential ultrafiltration consists of concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibers (Amicon, Millipore, Sartorius, Pall, GF, Sepracor). Within the scope of the invention, the use of membranes with a cut-off threshold below 1000 kDa, preferably between 100 kDa and 1000 kDa, or even more preferably between 100 kDa and 600 kDa, is advantageous.

As additional or alternative preprocessing steps size-exclusion chromatography, gel permeation chromatography or affinity chromatography might be carried out before or after contacting the biological sample with the capture surface.

To perform the gel permeation chromatography step, a support selected from silica, acrylamide, agarose, dextran, ethylene glycol-methacrylate copolymer or mixtures thereof, e.g., agarose-dextran mixtures, are preferably used. For example, such supports include, but are not limited to: SUPERDEX® 200HR (Pharmacia), TSK G6000 (TosoHaas) or SEPHACRYL® S (Pharmacia).

Affinity chromatography can be accomplished, for example, by using different supports, resins, beads, antibodies, aptamers, aptamer analogs, molecularly imprinted polymers, or other molecules known in the art that specifically target desired surface molecules on microvesicles.

Optionally, control particles may be added to the sample prior to microvesicle isolation or nucleic acid extraction to serve as an internal control to evaluate the efficiency or quality of microvesicle purification and/or nucleic acid extraction. These control particles include Q-beta bacteriophage, virus particles, or any other particle that contains control nucleic acids (e.g., at least one control target gene) that may be naturally occurring or engineered by recombinant DNA techniques. The control target gene can be quantified using real-time PCR analysis.

Preferably, the control particle is a Q-beta bacteriophage, referred to herein as "Q-beta particle." The Q-beta particle may be a naturally-occurring virus particle or may be a recombinant or engineered virus, in which at least one component of the virus particle (e.g., a portion of the genome or coat protein) is synthesized by recombinant DNA or molecular biology techniques known in the art. Q-beta is a member of the leviviridae family, characterized by a linear, single-stranded RNA genome that consists of 3 genes encoding four viral proteins: a coat protein, a maturation protein, a lysis protein, and RNA replicase. Due to its similar size to average microvesicles, Q-beta can be easily purified from a biological sample using the same purification methods used to isolate microvesicles, as described herein. In addition, the low complexity of the Q-beta viral single-stranded gene structure is advantageous for its use as a control in amplification-based nucleic acid assays. The Q-beta particle contains a control target gene or control target sequence to be detected or measured for the quantification of the amount of Q-beta particle in a sample. For example, the control target gene is the Q-beta coat protein gene. After addition of the Q-beta particles to the biological sample, the nucleic acids from the Q-beta particle are extracted along with the nucleic acids from the biological sample using the extraction methods and/or kits described herein. Detection of the Q-beta control target gene can be determined by RT-PCR analysis, for example, simultaneously with the biomarkers of interest (i.e., BRAF). A standard curve of at least 2, 3, or 4 known concentrations in 10-fold dilution of a control target gene can be used to determine copy number. The copy number detected and the quantity of Q-beta particle added can be compared to determine the quality of the isolation and/or extraction process.

Thus, 50, 100, 150, 200, 250, 300, 350, 400, 450, 500, 1,000 or 5,000 copies of Q-beta particles added to a bodily fluid sample. In a preferred embodiment, 100 copies of Q-beta particles are added to a bodily fluid sample. The copy number of Q-beta particles can be calculated based on the ability of the Q-beta bacteriophage to infect target cells. Thus, the copy number of Q-beta particles is correlated to the colony forming units of the Q-beta bacteriophage.

### Detection of nucleic acid biomarkers

For detection of RNA, the RNA is preferably reverse-transcribed into complementary DNA (cDNA) before further amplification. Such reverse transcription may be performed alone or in combination with an amplification step. One example of a method combining reverse transcription and amplification steps is reverse transcription polymerase chain reaction (RT-PCT), which may be further modified to be quantitative, e.g. quantitative RT-PCT as described in US Patent No. 5,639,606. Another example of the method comprises two separate steps: a first of reverse transcription to convert RNA into cDNA and a second step of quantifying the amount of cDNA using quantitative PCR.

RT-PCR analysis determines a Ct (cycle threshold) value for each reaction. In RT-PCR, a positive reaction is detected by accumulation of a fluorescence signal. The Ct value is defined as the number of cycles required for the fluorescent signal to cross the threshold (i.e., exceeds background level). Ct levels are inversely proportional to the amount of target nucleic acid, or control nucleic acid, in the sample (i.e., the lower the Ct level, the greater the amount of control nucleic acid in the sample). Alternatively, the copy number of the control nucleic acid can be measured using another art-recognized techniques.

The analysis of nucleic acids present in the isolated particles is quantitative and/or qualitative. For quantitative analysis, the amounts (expression levels), either relative or absolute, of specific nucleic acids of interest within the isolated particles are measured with methods known in the art. For qualitative analysis, the species of specific nucleic acids of interest within the isolated microvesicles are identified with methods known in the art.

### Working examples

### Inoculation preparation

Clostridium perfringens isolate being positive for netB and tpeL was removed from -80°C storage and aseptically streaked on trypticase soy agar plates containing 5% sheep blood (TSA II). Plates were incubated at 37°C overnight under anaerobic conditions. Fifteen hours prior to the start of the inoculum culture, an isolated colony from the overnight plate was inoculated into 50ml pre-reduced BHI broth and incubated at 37□C under anaerobic conditions for about 15hours. Then 50µl of the culture was added to 2 x 100 ml of pre-reduced BHI broth. The resulting broth-cultures were staggered so that the high dose and low dose reached their final concentrations at the same time (109 cfu /ml and 106cfu/ml respectively). Aliquots of each culture were taken at the end of the growth period and serially plated onto TSA II agar +5% sheep's blood to verify bacterial concentration.

### Animal experiment

Seven hundred and twenty (720) male broilers raised in 36 battery pens for 30 days. Birds were equally and randomly assigned (9 pens each) into one of four treatment groups (negative control, sham, low dose gavage and high dose gavage). The birds were fed with standard corn/soy feed without antimicrobials. On the 13th day of rearing, over or under-weight birds were removed from all pens and excluded from the experiment. The bird with normal weight range were then challenged orally with 1 ml of *C. perfringens* strain harbouring the netB plasmid and being positive for the Tpel gene succesively at days 14,15 and 16. The sham group was challenged with 1ml of PBS, whereas the negative control group was not subjected to any stress or pathogen challenge. Before the first inoculation (day 14), 3 birds from each pen were randomly selected, sacrificed for the collection of blood and digesta samples. Twelve hours after the second challenge, on day 15, 3 birds were again sacrificed from each pen for sample blood and digesta. The gut contents of all sacrificed birds were cultured overnight to determine the ratio of sporulated *C. perfringens* to the vegetative cells as previously described (Heikinheimo et al, 2004). To assess whether challenge was successful, PCR of *NetB* and *TpeL* genes of the gut contents was performed. At least 10 birds were left in each of the pens until the end of the trial. Excreta samples were collected at intervals until study was terminated on day 20 when all birds were sacrificed and three pooled blood samples were obtained from each group. GITs of birds were examined for NE typical signs/lesions and necropsies of the gastro-intestinal tract were taken from duodenum and jejunum for histopathological analysis. Broiler performance indicators like mortality rate, feed consumption and group pathology were determined (table 3).

### Sample collection

### 1. Fecal and Cecal Excreta Sample Collections

Sheets of paper were placed in the pens to collect pooled fecal and pooled cecal samples every 2 hours after the first challenge until 12 hours after the second inoculation (day 15). Subsequently, samples were collected every 4 hours until day 17. At the end of the trial on day 20, fecal and cecal samples were also collected. During any of the collection event at least 1 gram of fecal and cecal was obtained per pen, and samples were store at -80°C.

### 2. Blood Collection

Blood was collected directly from the heart using a 18 gauge needle. About 5-6 ml of whole blood was collected from 3 randomly selected birds per pen and transferred to collection tubes containing EDTA. Cells are removed from plasma by centrifugation for 10 minutes at 1,000-2,000 x g using a refrigerated centrifuge. Following centrifugation, the liquid component (plasma) was immediately transferred into a clean polypropylene tube and stored at -20°C.

### 3. Gut contents collection

Gut content was collected from a gut approximately 20 cm in length. Gut content was expelled and excised into PBS + 20% glycerol and immediately frozen at -20°C. Samples have then been used for quantitation of vegetative versus sporulated *C. perfringens* and PCR detection of netB and tpeL.

### Histology of chicken's intestine

### 1. Tissue collection and processing

A 5 cm section following the duodenal loop was removed from each bird and placed in 10% formalin, and the container was gently agitated to dislodge feed to allow formalin getting into the gut. After fixation, tissues were washed well in several changes of Phosphate Buffered Saline (PBS) and placed in 70% ethanol for storage. For the processing of tissues and infiltration of tissues with paraffin before making 5uM section, tissues were dehydrated by washing 1x for 2 min in 70% and 95% ethanol solutions successively, and then 2x for 2mins in 100% ethanol solution. Finally, tissues were rinsed 2x for 1 minute in Xylene before being embedded in paraffin. The processed tissue was then oriented in a mold, embedded in a paraffin block and cut in a microtome to generate sections of 5µM. Tissue slices are floated onto Poly-L-lysine (PLL) modified slides. The slides are dried and placed in an oven to "bake" the paraffin.

### 2. Staining of tissue sections with Harris Hematoxylin and Eosin.

In brief, sections were rehydrated by successively decreasing concentration of ethanol: 2x for 3min each in 100% ethanol, 2x for 3 mins each in 95% ethanol and 1x for 3 minutes in 70% ethanol. Slides were then rinsed for 5 minutes in distilled water and then stained for 6 minutes with Hematoxylin solution (Harris Hematoxylin, Sigma, HHS-32). Slides were rinsed in running tap water for about 20 minutes and decolorized in acid alcohol (95% Ethanol, 2578 ml dH₂O, 950ml HCL, 9ml) for 1-3 seconds. Slides were rinsed again with running tap water for 5 minutes then immersed in lithium carbonate for 3 seconds and then rinsed with tap water for 5 minutes. Tissues were counterstained with eosin working solution (100ml stock eosin (1% aqueous Eosin-Y and 1% aqueous Phloxin B), 10 ml stock Phloxin B, 780 ml 95% Ethanol, 4 ml glacial Acetic Acid) for 15 seconds and rehydrated by successive incubation in ethanol solutions (2 for 3 minutes each in 95% ethanol solution and 2x for 3minutes each in 100% ethanol solution). Finally, tissues were incubated in xylene solution for 5 minutes and then mounted with cytoseal for analysis.

### Detection of Clostridium perfingens netB and tpeL genes by PCR

**Table 1. primer pairs used for the detection of netB and tpeL genes in Clostridium perfringens isolates from chicken's intestine**

| **Primer ID** | **Sequence** | **Target Gene** | **Product Length** |
|---|---|---|---|
| netB5F | CGCTTCACATAAAGGTTGGAAGGC | *netB* | 316 |
| netB5R | TCCAGCACCAGCAGTTTTTCCT | *netB* | 316 |
| AKP80F | ATATAGAGTCAAGCAGTGGAG | *tpeL* | 466 |
| AKP81R | GGAATACCACTTGATATACCTG | *tpeL* | 466 |

### 1. DNA extraction and Quantification of DNA concentration

DNA extracted from *C*. *perfringens strains isolated* from intestinal contents was performed with commercial DNA extraction kits from Qiagen, Hilden, Germany, according to the manufacturer's instruction.

### 2. DNA amplification

PCR amplification was performed in a final volume of 25µl containing 1 ng of DNA template, 0.2µM of each of the primers for the gene to be amplified or detected and 12 µl of 1x PCR master mix consisting of 1.2 units/25 µL of Taq DNA polymerase. The amplification was carried out in a Gene amplification PCR system, 9600 Thermocycler (Master cycler eppendorf, Germany). Initial denaturation was at 95°C for 5 min., followed by 40 cycles for 95°C for 30 sec., annealing at 55°C for 30 sec and extension at 72°C for 30 sec, with a final extension at 72°C for 7 minutes and hold for 40°C.

### Sample processing

A total of 36 plasma samples, 96 fecal samples and 87 cecal samples were processed for isolation and extraction of microvesicle total RNA. Plasma volumes varied between 0.5 ml to 3 ml from the different animals.

### Plasma preparation

Briefly, blood was collected into K2 EDTA tubes and mixed well with complete inversions. Then the tubes were centrifuged at 1300 x g for 10 min to separate the blood cells and plasma. Then plasma was removed and filtered through a 0.8µm filter to remove cell debris and platelets. All plasma samples were then aliquoted into 1ml cryovials and stored at -80°C until use.

### Isolation of Microvesicles and RNA extraction

State of the art methods of extracting microvesicles and further purification of the contents of microvesicles (MV) from biomaterials such as blood, csf, tissues and cell cultures have been described previously in US6899863B1 (Dhellin et al., 2005), EP 2 495 025 A1, WO 2014107571 A and 20140178885A1. Methods include the use of high speed ultracentrifugation, or the use of low speed centrifugation to separate cell debris followed by size exclusion or affinity binding to specific membranes or filtration (see review by Witwer & Colleagues, 2013). Several Immunological methods of purifying microvesicles have also been described (Thery, et al., 2016; Clayton et al., 2001; Wubbolts et al., 2003). Microvesicles can be purified by the use of matrixes, beads or magnetic particles functionalized with antibodies binding to surface proteins of microvesicles to positively select a desired population or with antibodies binding to surface proteins of other cellular components for their depletion (Kim et al., 2012 and Mathivanan et al., 2010). Rapid methods of isolation of microvisicles available to research include the use of microfluidic devices (2010, Chen, et al., 2010; EP 2740536 A2) and commercial kits (e.g., SeraMir™ (System Biosciences), ExoSpin™ (Cell Guidance), ExoMir™ (Bioo Scientifics), miRCURY™ (Exiqon), requiring little or no specialized laboratory equipment.

Herein, extraction of microvesicles from plasma, fecal and cecal samples was performed by the methods described in WO2014/107571A1 (Enderle, *et al.,* 2014). The method involved the isolation of microvesicles from a biological fluid by capturing them on the surface of a Q functionalized membrane filter (Exo50, Exosome Diagnostics). The RNA content of the captured microvesicles was released by lysis with Qiazol, and then the RNA was purified by chloroform extraction or with commercial RNAesay kit from Qiagen. In brief, the plasma sample was allowed to thaw slowly on ice and diluted 1:1 with PBS or 0.9% NaOH; the plasma solution was centrifuged at 300 g for 10 minutes to remove cells and debris. The supernatant was passed through a 0.8µm filter and then the flow through was centrifuged at 20,000 g at 4°C for 30 minutes to remove any remaining cell debris. Exo50 column with affinity membrane that binds microvesicles was pre-conditioned by allowing 5 ml loading buffer (2X) 100 mM Phosphate Buffer, 370mM NaCl) to run through it. Then, the supernatant containing the microvesicles obtained from the second centrifugation step was spiked 1000 copies of Qbeta particle to examine the extraction and recovery efficiency upon loading onto the preconditioned spin column. The column was spun briefly and the flow-through was discarded. The column was washed twice with 2 ml Wash buffer (250mM Bis Tris Propane, pH6.5-7 and (IX) 50mM phosphate buffer, 185mM NaCl) and after the second wash, the column was spun briefly and the flow-through was discarded. Microvesicles bound to the membrane were lysed to release content by the addition of 5ml (2,25 ml) of lysis buffer (Qiazol) and spun to collect the lysate. RNA was extracted from the lysate with RNeasy kit, Qiagen, following the manufacture's instruction. The RNA eluted with RNase-free water was pooled for duplicated samples to obtain a total volume of 46µl per sample. 1 µL RNA was applied on Agilent RNA 6000 Nano Kit to assess the quality and concentration of the RNA, while the remaining RNA was stored at -80°C for further downstream use (QPCR or Sequencing). The extraction efficiency and quantity of RNA extraction was assessed by comparing the Q-beta control target gene (Q-beta coat protein gene) expression in the extracted RNA by RT-PCR analysis to those of the target genes of interest. A standard curve of at least 2, 3, or 4 known concentrations in 10-fold dilution of a control target gene was used to determine the copy number. The copy number detected and the quantity of Q-beta particle added was compared to determine the quality of the isolation and/or extraction process.

### Fecal & cecal Samples

Since plasma and cecal samples contain large coarse materials, additional preprocessing steps are applied before performing microvesicle isolation and RNA extraction using the Exo50 kit and associated methods as described above for plasma samples. For instance, a 200 mg of fecal sample was allowed to thaw slowly; then 1ml of cold PBS or 0.9% sterile NaOH was added to homogenize the fecal / cecal sample followed by vortexing for about 1 minute. The resulting suspension is diluted 1:1 with PBS and then centrifuged at 20,000g for 30 min at 4° C to remove large unwanted particles. Supernatant was filtered, through a 0.8 µm mixed cellulose ester filter (e.g., Millipore filter) and the filtrate was stored at 4°C until further use or processed as described for plasma sample above.

**Table 2. List of primers and probe used for the qPCR to quantify levels of expression of targets. The miRNA were purchased from Life Technologies and in house designed primers and probe were synthesized by IDT (Integrated DNA Technologies, USA)**

| **Target** | **Assay ID / Primers and probes (where applicable)** |
|---|---|
| miR-155 | TM:000479 (Life Technologies #4427975) |
| miR-16 | TM:000391 (Life Technologies #4427975) |
| miR-24 | TM:000402 (Life Technologies #4427975) |
| miR-206 | TM:000510 (Life Technologies #4427975) |
| virX (IDT) | Forward: 5'- TCGTATTCACTGTAGGAGAACAAG -3' |
| | Reverse: 5'- TGAGCTTTTCTTGCTTTTCTGC -3' |
| | Probe: 5'- /56-FAM/ ACGATCCAG /ZEN/ TTAGATGCCCAGCTTG/3IABkFQ/-3' |
| NetB (IDT) | Forward: 5'- TGGTGCTGGAATAAATGCTTCA -3' |
| | Reverse: 5'- ACCGTCCTTAGTCTCAACAA -3' |
| | Probe: 5'- /56-FAM/ TGATGCAAA /ZEN/ TTTAGCATCATGGGA/3IABkFQ/ -3' |
| SAM (IDT) SybrGreen assay | Forward: 5'- AAATAAGGAGTTGATAAACATGAAA -3' |
| | Reverse: 5'- TGTTACTGGTGAACAACAAAAAT -3' |
| colA (IDT) | Forward: 5'- TGAAAGAACACCAGAGGAAAGT -3' |
| | Reverse: 5'- CCTGCAAAGAACTCTGCTGT -3' |
| | Probe: 5'- /56-FAM/ TGATGCAAA /ZEN/ GTGGGGGCAAGGA /3IABkFQ/-3' |
| CPE0956 (IDT) SybrGreen assay | Forward: 5'- AATAGGATTTATAGCTGGAT -3' |
| | Reverse: 5'- AGTTAGTTTTCCAAGGATAG -3' |
| scRNA (IDT) SybrGreen assay | Forward: 5'- TATGTAAGTGGTGTTGAGAG -3' |
| | Reverse: 5'- ATGAAAATGACTGCTTATC -3' |

### QRT-PCR of fecal / cecal derived RNA

Reverse transcription of the fecal and cecal RNA was performed with the SuperScript VILO cDNA Synthesis Kit following the instructions of the manufacturer and the following modifications: the RT reaction consisted of 11µl (5x VILO Reaction Mix), 5.5µl (10x SS Enzyme Mix), 5.5µl (Nuclease free H20) and 33µl (RNA). While the RT cycle profile consisted of 4 sequential incubation cycles / holding at 25° for 10 min, 42° for 60 min, 85° for 5 min and a final holding step at 4° C.

### Q RT-PCR for Plasma and fecal/cecal derived miRNA:

Reverse transcription of the miRNA was performed in triplicate for each sample by using Reverse Transcription of RNA using TaqMan MicroRNA Reverse Transcription Kit with the following RT master mix recipe and cycling profile: RT reaction consisted of 6µl (RT primer pool), 0.3µl (dNTPs of 100mM), 1.5µl (10x RT buffer), 0.2µl RNase inhibitor, 3.0µl MultiScribe RT and 4µl (4 µl RNA for Fecal and cecal samples, and 2µl RNA + 2µl TE buffer for plasma samples). While the RT cycle profile consisted of 4 sequential incubation cycles / holdings at 16° for 30 min, 42° for 30 min, 85° for 5 min and a final holding step at 4°. The RT primer pool was derived by 10 microliter of each RT primer was pooled and diluted to 1 ml with TE buffer, making the final concentration of each RT primer 0.05X. Five times (5×) of the customized miRNA assay (miR-16, miR-21, miR-24, miR- 155 and miR-206) was added to the respective RT primer pool.

### QPCR of fecal / cecal RNA

qPCR was performed for probe-based assays for RNA genes using the Qiagen Rotor -Gene Q, according to manufacturer's instruction. The annealing temperature of virX, colA and netB assays was 57°C, while the annealing temperature for the analysis of 16s rRNA of *Clostridium perfringens* was 60°C. The master mix reactions for the assays consisted of 5.5µl (Nuclease free water), 10 µl (TaqMan Fast Universal PCR Master Mix no AmpErase UNG), 0.5µl forward primer (20uM), 1.5 µl reverse primer (20µM), 0.25µl probe (20µM), 0.25µl UNG(Uracil-N-glycosylase), and 2µl cDNA. For the amplification of 16s RNA, 1µl each of forward and reverse was used.

Rotor-Gene SYBR Green PCR Kit was performed for small non coding RNA VR-RNA, e45nt and scRNA. The master mix reactions for the assays consisted of 5.75µl (Nuclease free water), 10 µl (Rotor Gene SYBR 2x Master Mix), 0.5µl forward primer (20uM), 1.5 µl reverse primer (20µM), 0.25µl probe (20µM), 0.25µl UNG (Uracil-N-glycosylase), and 2µl cDNA. The amplification was performed with the following condition: First holding step at 50° C for 2 min, followed by another hold at 95o C for 10 and then 40 cycles consisting of 95° C for 2 seconds, 60o for 15 secs 72o C for 20 secs.

### Statistical analysis

All statistical calculations were performed using the statistics software R. As a result of assay's stoichiometry and overall efficiency of the analytical protocols, replacement of missing values (no signal) were imputed before normalization with 40, which is the highest possible CT value in a qPCR with 40 amplification cycles. Moderate statistics for pairwise comparison of the treatment groups were calculated with the ImFit function in the package limma (limma: linear models for microarray data, Smyth G.K. In: Bioinformatics and Computational Biology Solutions using R and Bioconductor, Springer, New York, pp 397-420, R package version 2.16.5) and the resulting p values were adjusted according to the method of Benjamini and Hochberg, (1995) to obtain the q values (false discovery rate)

### Multivariate Analysis (ANOVA)

The dependency between time, treatment and sample type was qualitatively examined. A formal quantitative assessment of possible associations was performed by running two-way ANOVA and non-parametric Kruskal-Wallis tests. Both methods have been applied independently to miRNA and bacterial RNA data. The two way ANOVA was used to test for two main effects and an interaction. The first main effect was time after treatment (in hours), the second main effect was the type of treatment (control, sham, high dose, low dose). The Kruskal-Wallis test was applied separately to time and treatment, to test for an association of these factors with gene expression. No multivariable interaction was assessed by this method. Three samples types (cecal, excreta and plasma) were analyzed for 4 miRNAs (miR-16, miR-24, miR-155 and miR-206) and a total of 216 duplicate data was available. Also six bacterial targets were analyzed in two sample types (cecal and excreta) and a total of 108 duplicate data was available. The means of the duplicate data (mean Ct value of each sample) were used for further statistical analyses. Analysis was performed on both raw data and normalized data. MiR-191 was used as normalizer for the microRNA analysis, whereas Ct values of 16S rRNA of *C. perfringens* were used as normarlizer for the bacterial targets. The anova lineal model (Anova (lm(expression∼time+treatment+time*treatment)) generated p values for three factors (time-effect, treatment-effect, interaction) for the normalized and raw data, while the 2 p values (time effect and treatment effect) were generated from the analyses with Kruskal-Wallis test for the normalized and raw data.

### Results:

**Table 3. Number of dead birds per group after C. perfringens challenge. One bird was dead in each of the groups: low-dose gavage group, the sham group, and the negative control group. 4 birds were dead in the group of birds challenged with high dose of Clostridium perfringens.**

| **Group** | **Initial group size** | **Birds sacrificed for bleeding days 14 & 15** | **Birds lost after C. perfringens inoculation** | **AFCR* - after challenge** | **Percent with typical gross pathology** | **No signs of performance loss and disease** |
|---|---|---|---|---|---|---|
| High-dose gavage | 180 | 54 | 4 | 1.60 | 50.70 | 49.30% |
| Low-dose gavage | 180 | 54 | 1 | 1.60 | 42.12 | 57.88% |
| Sham | 180 | 54 | 1 | 1.57 | 6.06^{b} | 93.94% |
| Negative control | 180 | 54 | 1 | 1.56 | 3.13 | 96.87% |
| P value | | | 0.200 | 0.746 | <0.001 | |

| | | | | | | |
|---|---|---|---|---|---|---|
| * AFCR (adjusted feed conversion ratio) determined as the amount of feed consumed divided by the birds body weight, corrected for mortalities and birds removed due to weight diversion from weight of birds before challenge | | | | | | |

**Table 4: Markers with significantly increased occurrence in membrane vesicles of cecal excrements**

| Marker | Raw FDR | Norm FDR |
|---|---|---|
| Sam region | 1,21 E-09 | 9,24E-02 |
| Swim zinc finger region | 9,52E-09 | 9,78E-02 |
| scRNA | 3,21 E-05 | 7,93E-03 |
| Mir206 | 8,55E-05 | 9,13E-04 |
| virX | 2,95E-04 | 5,93E-03 |
| netB | 1,04E-03 | 1,30E-03 |
| VR.RNA | 2,89E-03 | 3,21 E-05 |
| colA | 6,11 E-03 | 3,73E-04 |

**Table 5: Markers with significantly increased occurrence in membrane vesicles of fecal excrements**

| Marker | Raw FDR | Norm FDR |
|---|---|---|
| Sam region | 2,63E-11 | 8,18E-03 |
| scRNA | 8,94E-05 | 8,64E-04 |
| netB | 2,45E-04 | 2,75E-03 |
| virX | 3,24E-03 | 9,78E-02 |
| colA | 5,01 E-03 | 8,68E-04 |
| Swim zinc finger region | 5,81 E-02 | 2,26E-03 |
| VR.RNA | 1,36E-01 | 1,03E-04 |

**Table 6: Markers with significantly increased occurrence in membrane vesicles of blood samples**

| Marker | Norm FDR |
|---|---|
| Mir16 | 6,98E-03 |
| Mir155 | 1,56E-02 |
| Mir24 | 9,40E-02 |

### List of references

1. Thery C, Amigorena S, Raposo G, Clayton A (2006). Isolation and characterization of exosomes from cell culture supernatants and biological fluids. Curr Protoc Cell Biol., Chapter 3: Unit 3.22.
2. Clayton A, Court J, Navabi H, Adams M, Mason MD, Hobot JA, et al. (2001). Analysis of antigen presenting cell derived exosomes, based on immuno-magnetic isolation and flow cytometry. J Immunol. Methods . 2001; 247:163-74.
3. Wubbolts R, Leckie RS, Veenhuizen PT, Schwarzmann G, Mobius W, Hoernschemeyer J, et al. (2003). Proteomic and biochemical analyses of human B cell-derived exosomes. Potential implications for their function and multivesicular body formation. J Biol Chem., 278:10963-72.
4. Kim G, Yoo CE, Kim M, Kang HJ, Park D, Lee M, et al, (2012) Noble polymeric surface conjugated with zwitterionic moieties and antibodies for the isolation of exosomes from human serum. Bioconjug. Chem., 23:2114-20.
5. Mathivanan S, Lim JW, Tauro BJ, Ji H, Moritz RL, Simpson RJ (2010). Proteomics analysis of A33 immunoaffinity-purified exosomes released from the human colon tumor cell line LIM1215 reveals a tissue-specific protein signature. Mol. Cell. Proteomics, 9: 197-208.
6. Smyth G.K (2004) linear models for microarray data In: Bioinformatics and Computational Biology Solutions using R and Bioconductor, Springer, New York, pp 397-420
7. Hochberg and Roy (1995) Controlling the false discovery rate: a practical and powerful approach to multiple testing. Journal of the Royal Statistical Soc. Ser B (Methodological), 57:289-300
8. Heikinheimo, A, and Lindstro¨m,M, and Korkeala, H. (2004) Enumeration and Isolation of cpe-Positive Clostridium perfringens Spores from Feces. Journal of Clinical Microbiology, 42, 9: 3992-3997
9. Dinh, H., et al., Modulation of microRNAs in two genetically disparate chicken lines showing different necrotic enteritis disease susceptibility. Vet. Immunol. Immunopathol. (2014), http://dx.doi.org/10.1016/j.vetimm.2014.02.003
10. Aade, U.P., and Wankhede, H.J, and Kaldate, K.D (2012). Haematological parameters change in Gallus gallus domesticus infected with cestode parasite. International Multidisciplinary Research Journal, 2(4):13-15
11. Chuku LC et al. (2012) A comparison of the haematological and biochemical indices of broiler and red jungle (hamburgh) fowl (gallus gallus domesticus), Discovery Nature, 2012, 1(1), 15-18,
12. Wang et al. (2012) Integrated analysis of microRNA expression and mRNA transcriptome in lungs of avian influenza virus infected broilers BMC Genomics 2012, 13:278
13. Jiang et al. (2014) Membrane vesicles of Clostridium perfringens type A strains induceinnate and adaptive immunity. International Journal of Medical Microbiology 304 (2014) 431-443
14. Obana, et al. (2013) Structural Requirement in Clostridium perfringens Collagenase mRNA 5'Leader Sequence for Translational Induction through Small RNA-mRNA Base Pairing Journal of Bacteriology , Volume 195 Number 12 p. 2937-2946
15. Ohtani et al. (2002). Identification of a novel locus that regulates expression of toxin genes in Clostridium perfringens FEMS Microbiology Letters, 209: 113-118
16. Nakamura et al. (1995) Small cytoplasmic RNA (scRNA) gene from Clostridium perfringens can replace the gene for the Bacillus subtilis scRNA in both growth and sporulationMicrobiology (1995), 141: 2965-2975
17. KEYBURN et al. (2010) Association between avian necrotic enteritis and Clostridium perfringens strains expressing NetB toxin Vet. Res. 41:21
18. Shojadoost et al. (2012). The successful experimental induction of necrotic enteritis in chickens by Clostridium perfringens: a critical review Veterinary Research, 43:74
19. Koga et al. (2011) Exosome can prevent RNase from degrading microRNA in feces. J Gastrointest Oncol 2011; 2: 215-222.
20. Saleem, Gulbeena (2013) Identification of biochemical markers for sub-clinical necrotic enteritis in broiler chickens (In Necrotic enteritis, disease induction, predisposing factors and novel biochemical markers in broilers chickens), Chapter 8, PhD thesis, University of Glasgow, UK.
21. Garcia et al. (2009). Experimental infection of commercial layers using a Salmonella enterica serovar Gallinarum strain: Leukogram and serum acute-phase protein concentrations. Revista Brasileira de Ciência Avícola 11, 263-270.
22. Piercy (1979). Acute phase responses to experimental salmonellosis in calves and colibacillosis in chickens: serum iron and caeruloplasmin. Journal of Comparative Pathology 89, 309-319.
23. Witwer et al. (2013) Standardization of sample collection, isolation and analysis methods in extracellular vesicle research Journal of Extracellular Vesicles, 2: 20360
24. Nilsson, et al. (2009) Prostate cancer-derived urine exosomes: a novel approach tobiomarkers for prostate cancer. Br J Cancer, 100:1603-7.
25. Li et al. (2009) Claudin-containing exosomes in the peripheral circulation of women with ovarian cancer. BMC Cancer, 9:244.
26. Shao et al. (2012) Protein typing of circulating microvesicles allows real time monitoring of glioblastoma therapy. Nat Med. , 18:1835-40.
27. Taylor and Gercel-Taylor Taylor (2008) MicroRNA signatures of tumor-derived exosomes as diagnostic biomarkers of ovarian cancer Gynecologic Oncology, 110 : 13-21
28. Cheruvanky et al. (2007) Rapid isolation of urinary exosomal biomarkers using a nanomembrane ultrafiltration concentrator. Am J Physiol Renal Physiol., 292: F1657-61.
29. Miranda et al.(2010) Nucleic acids within urinary exosomes/ microvesicles are potential biomarkers for renal disease. Kidney Int., 78:191-9.
30. Chen et al. (2010) Microfluidic isolation and transcriptome analysis of serum microvesicles. Lab Chip., 10:505-11.
31. Skog et al. (2008). Glioblastoma microvesicles transport RNA and proteins that promote tumour growth and provide diagnostic biomarkers. Nat Cell Biol., 10:1470-6.
32. Moore et al. (2008). Clostridium toxin netB (WO 2008/148166)
33. Dhellin et al. (2005). Method for preparing membrane vesicles (US6,899,863B1)
34. Lamparski et al. (2004) Method of producing membrane vesicles (US6,812,023B1)
35. Abrignani et al. (2007) Method for the preparation of purified HCV RNA by exosome separation (US7,198,923B1)
36. Giorgio (2012) Filter for the removal of micro-vesicles from biological fluids, methods anddevices using such a filter (EP 2495025 A1)
37. Enderle et al (2014) methods for isolating microvesicles (WO2014/107571)
38. Kyung-Hee et al. (2014) Composition and kit for diagnosing breast cancer including polynucleotide within vesicle, and method of diagnosing breast cancer using the same (US 20140178885A1)
39. Min Soak and Jong-Myeon (2014) Microfluidic apparatus and method of enriching target material in biological sample by using the same (EP2740536 A2)

## Claims

1. A method of detecting avian necrotic enteritis, in particular sub-clinical avian necrotic enteritis, wherein detection of necrotic enteritis is carried out by detecting the amount of at least one of the following sequences in an avian sample:
a) polynucleotides which have a sequence identity of at least 95 %, preferably 100 %, to a polynucleotide which consists of at least 40, preferably at least 60, 100, 150, 180 or 200, consecutive nucleotides of the nucleotide sequence as depicted in SEQ ID NO: 1,
b) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 3,
c) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 4;
d) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 7;
e) polynucleotides which have a sequence identity of at least 95 %, preferably 100 %, to a polynucleotide which consists of at least 20, preferably at least 25, 40, 60, 100, 150, 180 or 200, consecutive nucleotides of the nucleotide sequence as depicted in SEQ ID NO: 9;
f) polynucleotides which have a sequence identity of at least 95 %, preferably 100 %, to a polynucleotide which consists of at least 8 or 10, preferably at least 12, 14, 16, 18, 20, 25, 40, 60 or 80, consecutive nucleotides of the nucleotide sequence as depicted in SEQ ID NO: 10;
g) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 11;
h) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 12;
i) polynucleotides which have a sequence identity of at least 95 %, preferably 100 %, to a polynucleotide which consists of at least 8 or 10, preferably at least 12, 14, 16, 18, 20, 25, 40 or 60, consecutive nucleotides of the nucleotide sequence as depicted in SEQ ID NO: 13;
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

2. The method according to claim 1, wherein the avian sample is a bodily fluid, preferably blood, in particular blood plasma or blood serum, or a bodily excrement, preferably a fecal or cecal excrement.

3. The method according to claim 1 or 2, wherein the avian sample is a sample of poultry, preferably a sample of chickens, turkeys, ducks, geese, peacocks, pheasants, partridges, guinea fowl, quails, capercaillies, grouse, pigeons, swans, ostriches and parrots, most preferably chickens.

## Patentansprüche

1. Verfahren zum Nachweis nekrotischer Enteritis bei Vögeln, insbesondere subklinischer nekrotischer Enteritis bei Vögeln, wobei der Nachweis von nekrotischer Enteritis durchgeführt wird, indem die Menge wenigstens einer der folgenden Sequenzen in einer Vogelprobe ermittelt wird:
a) Polynukleotide, die eine Sequenzidentität von wenigstens 95 %, bevorzugt 100 %, zu einem Polynukleotid aufweisen, das aus wenigstens 40, bevorzugt wenigstens 60, 100, 150, 180 oder 200, aufeinander folgenden Nukleotiden der Nukleotidsequenz gemäß SEQ ID NO: 1 besteht,
b) Polynukleotide, die eine Sequenzidentität von wenigstens 95 %, bevorzugt 100 %, zu einem Polynukleotid aufweisen, das aus wenigstens 20, bevorzugt wenigstens 25, 40, 60, 100, 150, 180 oder 200, aufeinander folgenden Nukleotiden der Nukleotidsequenz gemäß SEQ ID NO: 3 besteht,
c) Polynukleotide, die eine Sequenzidentität von wenigstens 95 %, bevorzugt 100 %, zu einem Polynukleotid aufweisen, das aus wenigstens 20, bevorzugt wenigstens 25, 40, 60, 100, 150, 180 oder 200, aufeinander folgenden Nukleotiden der Nukleotidsequenz gemäß SEQ ID NO: 4 besteht;
d) Polynukleotide, die eine Sequenzidentität von wenigstens 95 %, bevorzugt 100 %, zu einem Polynukleotid aufweisen, das aus wenigstens 20, bevorzugt wenigstens 25, 40, 60, 100, 150, 180 oder 200, aufeinander folgenden Nukleotiden der Nukleotidsequenz gemäß SEQ ID NO: 7 besteht;
e) Polynukleotide, die eine Sequenzidentität von wenigstens 95 %, bevorzugt 100 %, zu einem Polynukleotid aufweisen, das aus wenigstens 20, bevorzugt wenigstens 25, 40, 60, 100, 150, 180 oder 200, aufeinander folgenden Nukleotiden der Nukleotidsequenz gemäß SEQ ID NO: 9 besteht;
f) Polynukleotide, die eine Sequenzidentität von wenigstens 95 %, bevorzugt 100 %, zu einem Polynukleotid aufweisen, das aus wenigstens 8 oder 10, bevorzugt wenigstens 12, 14, 16, 18, 20, 25, 40, 60 oder 80, aufeinander folgenden Nukleotiden der Nukleotidsequenz gemäß SEQ ID NO: 10 besteht;
g) Polynukleotide, die eine Sequenzidentität von wenigstens 95 %, bevorzugt 100 %, zu einem Polynukleotid aufweisen, das aus wenigstens 8 oder 10, bevorzugt wenigstens 12, 14, 16, 18, 20, 25, 40 oder 60, aufeinander folgenden Nukleotiden der Nukleotidsequenz gemäß SEQ ID NO: 11 besteht;
h) Polynukleotide, die eine Sequenzidentität von wenigstens 95 %, bevorzugt 100 %, zu einem Polynukleotid aufweisen, das aus wenigstens 8 oder 10, bevorzugt wenigstens 12, 14, 16, 18, 20, 25, 40 oder 60, aufeinander folgenden Nukleotiden der Nukleotidsequenz gemäß SEQ ID NO: 12 besteht;
i) Polynukleotide, die eine Sequenzidentität von wenigstens 95 %, bevorzugt 100 %, zu einem Polynukleotid aufweisen, das aus wenigstens 8 oder 10, bevorzugt wenigstens 12, 14, 16, 18, 20, 25, 40 oder 60, aufeinander folgenden Nukleotiden der Nukleotidsequenz gemäß SEQ ID NO: 13 besteht;
j) Polynukleotide (DNAs und RNAs), die zu den Sequenzen unter (a) bis (i) komplementär sind;
k) Polynukleotide (DNAs und RNAs), die zu den Sequenzen unter (j) komplementär sind;
l) Polynukleotide, die die Polynukleotide von (a) bis (k) umfassen.

2. Verfahren nach Anspruch 1, wobei es sich bei der Vogelprobe um eine Körperflüssigkeit, bevorzugt Blut, insbesondere Blutplasma oder Blutserum, oder Körperexkrement, bevorzugt ein Fäkal- oder Zäkalexkrement, handelt.

3. Verfahren nach Anspruch 1 oder 2, wobei es sich bei der Vogelprobe um eine Probe von Geflügel, bevorzugt eine Probe von Hühnern, Puten, Enten, Gänsen, Pfauen, Fasanen, Rebhühnern, Perlhühnern, Wachteln, Auerhühnern, Moorhühnern, Tauben, Schwänen, Straußen und Papageien, ganz besonders bevorzugt Hühnern, handelt.

## Revendications

1. Procédé de détection d'une entérite nécrotique aviaire, en particulier une entérite nécrotique aviaire sub-clinique, dans lequel la détection d'une entérite nécrotique est effectuée en détectant dans un échantillon aviaire la quantité d'au moins l'une des séquences suivantes :
a) polynucléotides qui ont une identité de séquence d'au moins 95%, de préférence 100%, avec un polynucléotide qui est constitué par au moins 40, de préférence au moins 60, 100, 150, 180 ou 200, nucléotides consécutifs de la séquence nucléotidique telle qu'illustrée dans la SEQ ID n° : 1,
b) polynucléotides qui ont une identité de séquence d'au moins 95%, de préférence 100%, avec un polynucléotide qui est constitué par au moins 20, de préférence au moins 25, 40, 60, 100, 150, 180 ou 200, nucléotides consécutifs de la séquence nucléotidique telle qu'illustrée dans la SEQ ID n° : 3,
c) polynucléotides qui ont une identité de séquence d'au moins 95%, de préférence 100%, avec un polynucléotide qui est constitué par au moins 20, de préférence au moins 25, 40, 60, 100, 150, 180 ou 200, nucléotides consécutifs de la séquence nucléotidique telle qu'illustrée dans la SEQ ID n° : 4 ;
d) polynucléotides qui ont une identité de séquence d'au moins 95%, de préférence 100%, avec un polynucléotide qui est constitué par au moins 20, de préférence au moins 25, 40, 60, 100, 150, 180 ou 200, nucléotides consécutifs de la séquence nucléotidique telle qu'illustrée dans la SEQ ID n° : 7 ;
e) polynucléotides qui ont une identité de séquence d'au moins 95%, de préférence 100%, avec un polynucléotide qui est constitué par au moins 20, de préférence au moins 25, 40, 60, 100, 150, 180 ou 200, nucléotides consécutifs de la séquence nucléotidique telle qu'illustrée dans la SEQ ID n° : 9 ;
f) polynucléotides qui ont une identité de séquence d'au moins 95%, de préférence 100%, avec un polynucléotide qui est constitué par au moins 8 ou 10, de préférence au moins 12, 14, 16, 18, 20, 25, 40, 60 ou 80, nucléotides consécutifs de la séquence nucléotidique telle qu'illustrée dans la SEQ ID n° : 10 ;
g) polynucléotides qui ont une identité de séquence d'au moins 95%, de préférence 100%, avec un polynucléotide qui est constitué par au moins 8 ou 10, de préférence au moins 12, 14, 16, 18, 20, 25, 40 ou 60, nucléotides consécutifs de la séquence nucléotidique telle qu'illustrée dans la SEQ ID n° : 11 ;
h) polynucléotides qui ont une identité de séquence d'au moins 95%, de préférence 100%, avec un polynucléotide qui est constitué par au moins 8 ou 10, de préférence au moins 12, 14, 16, 18, 20, 25, 40 ou 60, nucléotides consécutifs de la séquence nucléotidique telle qu'illustrée dans la SEQ ID n° : 12 ;
i) polynucléotides qui ont une identité de séquence d'au moins 95%, de préférence 100%, avec un polynucléotide qui est constitué par au moins 8 ou 10, de préférence au moins 12, 14, 16, 18, 20, 25, 40 ou 60, nucléotides consécutifs de la séquence nucléotidique telle qu'illustrée dans la SEQ ID n° : 13 ;
j) polynucléotides (ADN et ARN) qui sont complémentaires aux séquences selon les paragraphes (a) à (i) ;
k) polynucléotides (ADN et ARN) qui sont complémentaires aux séquences selon le paragraphe (j) ;
l) polynucléotides qui comprennent les polynucléotides selon les paragraphes (a) à (k).

2. Procédé selon la revendication 1, dans lequel l'échantillon aviaire est un liquide corporel, de préférence du sang, en particulier du plasma sanguin ou du sérum sanguin, ou un excrément corporel, de préférence un excrément fécal ou cæcal.

3. Procédé selon la revendication 1 ou la 2, dans lequel l'échantillon aviaire est un échantillon de volaille, de préférence un échantillon de poulets, dindes, canards, oies, paons, faisans, perdreaux, pintades, cailles, coqs de bruyère, grouse, pigeons, cygnes, autruches et perroquets, de manière préférée entre toutes des poulets.
